(19) **Europäisches Patentamt / European Patent Office / Office européen des brevets**

(11) **EP 4 671 275 A1**

(12) **EUROPEAN PATENT APPLICATION**
published in accordance with Art. 153(4) EPC

(43) Date of publication:
**31.12.2025 Bulletin 2026/01**

(21) Application number: **24760689.0**

(22) Date of filing: **20.02.2024**

(51) International Patent Classification (IPC):
*C07K 16/26* (2006.01)       *C12N 9/16* (2006.01)
*A61K 38/46* (2006.01)       *A61K 39/395* (2006.01)
*A61P 3/00* (2006.01)       *A61P 25/28* (2006.01)

(52) Cooperative Patent Classification (CPC):
A61K 38/46; A61 39/395; A61P 3/00;
A61P 25/28; C07K 16/26; C12N 9/16

(86) International application number:
**PCT/RU2024/050043**

(87) International publication number:
**WO 2024/177535 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.02.2023 RU 2023104014**

(71) Applicant: **Lithium Holding L.L.C-FZ**
**Nad Al Sheba Dubai (AE)**

(72) Inventors:
• **SHUKUROV, Rakhim Rakhmankulyyevich**
**Moscow, 107370 (RU)**

• **RESHETNIK, Elizaveta Vyacheslavovna**
**Moscow, 115583 (RU)**
• **KHAMITOV, Ravil Avgatovich**
**Moscow, 125466 (RU)**
• **SHUSTER, Aleksandr Mikhailovich**
**Moscow, 115184 (RU)**

(74) Representative: **Boskovic, Davor**
**Producta d.o.o.**
**Zinke Kunc 3a**
**10000 Zagreb (HR)**

Remarks:
The complete document including Reference Table(s) and the Sequence Listing(s) can be downloaded from the EPO website

(54) **PHARMACEUTICAL COMPOSITION AND METHOD FOR THE PROPHYLAXIS AND TREATMENT OF A LYSOSOMAL ENZYME DEFICIENCY IN A SUBJECT WITH MUCOPOLYSACCHARIDOSIS TYPE II**

(57) The invention relates to the field of biotechnology, and more particularly to a pharmaceutical composition and a method for the prophylaxis and treatment of a lysosomal enzyme deficiency in a subject, which can be used in medicine. The present invention relates to an HIR-Fab-IDS compound that can be used for the prophylaxis or treatment of a lysosomal enzyme deficiency in a subject suffering from a lysosomal storage disease in the form of mucopolysaccharidosis type II (MPS II), wherein at least one dose of said HIR-Fab-IDS compound is administered to the subject in an amount of from 1 to 12 mg/kg, and further relates to a pharmaceutical composition for use in the prophylaxis or treatment of a lysosomal enzyme deficiency in a subject with the lysosomal storage disease MPS II, wherein the composition contains said HIR-Fab-IDS compound, as well as to a method for the prophylaxis or treatment of a lysosomal enzyme deficiency in a subject with the lysosomal storage disease MPS II, which includes administering at least one dose of said HIR-Fab-IDS compound to the patient.

EP 4 671 275 A1

**Description**

**Field of art**

The invention relates to the field of biotechnology, specifically to a pharmaceutical composition and a method for the prophylaxis and treatment of treating lysosomal enzyme deficiency in a subject, which can be used in medicine. The present invention relates to an HIR-Fab-IDS compound used for the prophylaxisprophylaxis or treatment of a lysosomal enzyme deficiency in a subject suffering from a lysosomal storage disease in the from of mucopolysaccharidosis type II, wherein at least one dose of the HIR-Fab-IDS compound is administered to the subject in an amount from 1 to 12 mg/kg, and to a pharmaceutical composition for use in the prophylaxisprophylaxis or treatment of a lysosomal enzyme deficiency in a subject having a lysosomal storage disease which is mucopolysaccharidosis type II, wherein the composition comprises the HIR-Fab-IDS compound, as well as to a method for the prophylaxisprophylaxis or treatment of a lysosomal enzyme deficiency in a subject with a lysosomal storage disease which is mucopolysaccharidosis type II, comprising administering at least one dose of the HIR-Fab-IDS compound to the patient.

**Prior art**

The use of enzymes in therapy has long been known from the state of the art. Modern medicine increasingly uses therapeutic enzymes in a variety of fields of medicine due to their high activity and specificity. Currently, the following areas of enzyme therapy have emerged (Kazanskaya N. F. et al., 1984): 1) elimination of enzyme deficiency in order to compensate for congenital or acquired functional insufficiency; 2) removal of non-viable, denatured structures, cellular and tissue fragments; 3) lysis of thrombi; 4) complex therapy of malignant neoplasms; 5) detoxification of the body.

The use of therapeutic enzymes to eliminate enzyme deficiency in order to compensate for congenital or acquired functional insufficiency has been practiced for a long time. Treatment of congenital enzyme deficiencies, of which more than 150 have been described, is an important problem in replacement therapy. Such hereditary diseases as glycogenoses, lipidoses, mucopolysaccharidoses and other lysosomal storage diseases are treated primarily by intravenous administration of recombinant analogues of the corresponding native enzymes.

Lysosomal storage diseases are a group of rare (orphan) hereditary metabolic diseases caused by the absence or deficiency of lysosomal enzymes involved in the breakdown of complex molecules.

Currently (Novikov P. V., 2014) the following groups of lysosomal storage diseases are distinguished: 1) mucopolysaccharidoses; 2) lipidoses (sphingolipidoses: GM1- and GM2-gangliosidoses, Gaucher disease, galactosialidosis, Farber granulomatosis, leukodystrophies, Niemann-Pick disease types A and B, etc.); 3) mucolipidoses, 4) glycoproteinoses (fucosidosis, sialidosis, mannosidosis, glycogen storage disease type II: Pompe disease, Danon disease, etc.): 5) neuronal ceroid lipofuscinoses: 6) other storage diseases (Niemann-Pick disease type C, Wolman disease, cholesterol storage disease, cystinosis, Sal's disease, pycnodysostosis, etc.).

Mucopolysaccharidoses (MPS) are a group of nine (I-IX) or fourteen (with intermediate forms) metabolic diseases caused by more than 40 genetic disorders leading to the absence or functional insufficiency of a number of lysosomal enzymes involved in the hydrolytic breakdown of glycosaminoglycans (mucopolysaccharides). Glycosaminoglycans are oligosaccharides involved in the formation of bones, cartilage, ligaments, cornea, skin, connective tissue and synovial fluid (Burrow T. A. et al, 2013).

Patients suffering from mucopolysaccharidosis have a deficiency or absence of at least one of 11 enzymes of glycosaminoglycan catabolism, which over time leads to a gradual accumulation of these carbohydrates in cells, body fluids, including connective tissue. As a result, the permanent accumulation of mucopolysaccharides leads to cell damage, tissue and organ dysfunction, most often manifested in hypertension-hydrocephalic syndrome, hepatosplenomegaly, cardiovascular failure, bone and joint complications, functional disorders of the central nervous system (CIS) up to severe cognitive impairment and dementia (Table 1) (Burrow T. A. et al, 2013).

Table 1. Characteristics of various MPS syndromes

| Syndrome | Enzyme defect | Defective chromosome, inheritance type | Clinical presentation, laboratory data | Neurological component |
|---|---|---|---|---|
| Hurler (MPS-I-H) | Alpha-L-iduronidase | Chromosome 4, autosomal recessive type | Children die at the age of 6-10 years from cardiac and pulmonary decompensation. Elevated levels of heparan and dermatan sulfates in urine (Connock et al. 2006). | Yes |

(continued)

| Syndrome | Enzyme defect | Defective chromosome, inheritance type | Clinical presentation, laboratory data | Neurological component |
|---|---|---|---|---|
| Scheie (MPS-I-S) | Alpha-L-iduronidase (incomplete) | Chromosome 4, autosomal recessive type | Mild variant, normal intelligence, normal stature. Patients live up to 30 years. Elevated levels of heparan and dermatan sulfates in urine (Connock et al. 2006, Pastores et al. 2007). | No |
| Hunter (MPS II) | Iduronate-2-sulfatase | X-linked recessive type | Boys suffer. Mental development is reduced. Patients live up to 30-40 years. There is no corneal opacity. There is no dorsolubial hump hump. Progressive deafness and hirsutism are characteristic. Elevated levels of heparan and dermatan sulfates in urine (Wraith et al. 2008, da Silva et al. 2016, Burrow and Leslie 2008). | Yes |
| Sanfilippo (MPS-III) | Type A - sulfamidase. Type B - b-N-acetyl-glucosaminidase. Type C - acetyl-CoA-glucosamini-N-acetyltransferase. Type D - alpha-N-acetylglucosamine-6-phosphatase | Chromosomes 12 and 17, autosomal recessive type | Severe retardation of intelligence, macrocephaly, very aggressive behavior. Relatively weak skeletal changes. Visceromegaly appears at school age. Clinically, the types cannot be differentiated. Heparan sulfate in urine (Valstar et al. 2008). | Yes |
| Morquio (MPS-IV) | N-acetylgalactosamine-6-sulfatase (type A) and beta-galactosidase (type B) | Chromosomes 3 and 16, autosomal recessive type | Normal intelligence. Severe skeletal deformity. Corneal opacity. Lesser degree of CNS damage. The first clinical signs appear at the age of 2-3 years. Die at the age of 20-35 years. Keratan sulfate in urine (Northover, Cowie and Wraith 1996). | No |
| Maroteaux-Lamy (MPS-VI) | N-acetyl-galactosamine-4-sulfatase | Chromosome 5, autosomal recessive type | Mild growth retardation, prominent hump, normal intelligence, mild facial abnormalities. Dermatan sulfate in urine (Garrido et al. 2008). | No |
| Sly (MPS-VII) | Beta-glucuronidase | Chromosome 7, autosomal recessive type | Varies in mental development, prominent hump, hepatosplenomegaly, mild facial abnormalities. Dermatan sulfate in urine. | Yes |

Mucopolysaccharidosis type I is caused by a deficiency of the lysosomal enzyme, $\alpha$-L-iduroidase. This deficiency results in the accumulation of mucopolysaccharides, especially dermatan sulfate, in tissues and organs. The accumulation of excess dermatan sulfate leads to the gradual development of numerous morphological abnormalities in tissues and organs. Mucopolysaccharidosis type I is characterized by an autosomal recessive type of inheritance.

To date, two effective treatment methods for MPS type I have been developed: hematopoietic stem cell transplantation (HSCT) and enzyme replacement therapy (ERT).

HSCT is used to treat only severe forms of MPS type I: Hurler syndrome, which allows for correction of the deficiency of the enzyme alpha-L-iduronidase and, in turn, leads to a significant improvement in the patient's condition, although some severe complications of the disease do not regress completely. HSCT should be performed as early as possible, before the onset of severe neurological disorders. Despite the improvement in the patient's condition, HSCT is accompanied by a high risk of serious post-transplant complications and is a complex, multistage, high-cost procedure.

ERT is safe, well tolerated by patients, does not cause severe adverse effects and leads to the decomposition of non-hydrolyzed substrate. Possible rare reactions to the administration of the drug are due to the formation of antibodies against the administered protein, but they are not constant and, as a rule, are quickly stopped by standard drugs. The principle of ERT is based on the restoration of the level of enzymatic activity sufficient for the hydrolysis of accumulated substrates and for the prophylaxis of their further accumulation.

Mucopolysaccharidosis type II (MPS II, Hunter syndrome), unlike all other forms of mucopolysaccharidoses, which have an autosomal recessive type of inheritance, is the only form of mucopolysaccharidoses with an X-linked recessive type of inheritance. MPS II is a progressive pathology of lysosomal storage, heterogeneous in clinical manifestations, arising due to the deficiency of the enzyme iduronate-2-sulfatase (idursulfase). This enzyme is normally involved in the degradation of the mucopolysaccharides dermatan sulfate and heparan sulfate due to the hydrolytic cleavage of the O-linked sulfate group. Accordingly, in the case of MPS II, the main pathogenetic mechanism is associated with the progressive accumulation of dermatan and heparan sulfates in lysosomes.

The most common clinical symptoms of MPS II are mental retardation, enlarged tongue, characteristic facial skeletal abnormalities, alopecia, dental malformations, restrictive lung disease, hepatosplenomegaly, heart valve abnormalities, bone and joint abnormalities, and severe short stature. Progressive neurological disorders accompanied by hydrocephalus and increased intracranial pressure are also common. Fatal outcome usually occurs during the second or third decade of life, most often due to respiratory and/or cardiac failure. It is important to emphasize that the disease progresses with involvement of the nervous system in the pathological process, including decreased intelligence.

Today, there are two fundamental methods of treating MPS II - ERT and symptomatic therapy (includes the use of hepatoprotectors, cardiovascular and anti-inflammatory drugs, vitamins and drugs that improve antioxidant protection). HSCT for the treatment of MPS II, unlike mucopolysaccharidosis type I, is not effective.

Elaprase® (INN: idursulfase) (Shire, USA) is a medical product representing recombinant human iduronate-2-sulfatase. This enzyme is responsible for the hydrolysis of C2-sulfate ester bonds of iduronic acid residues, which are part of the glucosaminoglycans (mucopolysaccharides) dermatan sulfate and heparan sulfate (Burrow T. A. el al., 2013). The usual regimen for the administration of Elaprase® is 0.5 mg/kg of body weight, once a week; administration is carried out by intravenous infusion for 3 hours (the infusion time can be gradually reduced to 1 hour).

Idursulfase contains two disulfide bonds and eight N-linked glycosylation sites occupied by complex high mannose oligosaccharides. The presence of M6P residues in the oligosaccharide chains allows the recombinant enzyme to bind to M6P receptors on the surface of target cells, which leads to the internalization of this enzyme into cells and its internalization into lysosomes, where it ensures the degradation of lysosomal mucopolysaccharides (Muenzer J., et al., Genet. Med. 2006 Aug; 8(8): 465-473).

When conducting ERT, the life expectancy of patients with Hunter syndrome increases several times. Despite this, Elaprase® also does not penetrate the blood-brain barrier, as a result of which, when using the ERT Elaprase®, patients die at the age of 20 from neurodegenerative consequences, while in the second decade of life, patients receiving Elaprase®, as a rule, experience great difficulties in learning and need assistants even in everyday life (da Silva E. M. et al., 2016; Wraith J.E. et al., 2008).

Thus, a common drawback of all currently existing drugs used for ERT of mucopolysaccharidoses types I and II is their inability to penetrate the blood-brain barrier into the central nervous system, which limits the effectiveness of these drugs in patients with nervous system damage associated with mucopolysaccharidosis, including mucopolysaccharidosis types I and II.

In many lysosomal storage diseases, there is a need for improved internalization of the enzyme into the cells of certain peripheral tissues (diaphragm muscles in Pompe disease, liver and spleen in Hunter disease, kidneys in Fabry disease, etc.) (Hawkes C. et al., 2004).

Thus, the task of developing a therapeutic compound, a pharmaceutical composition and a method for prophylaxis or treatment that could be used to treat a lysosomal storage disease such as MPS type II and that would retain the functional properties of the corresponding therapeutic enzyme, while demonstrating high activity and improved ability to be transported to lysosomes of tissue cells of various organs, including lysosomes of nervous tissue cells, and would allow for a significant improvement in the quality and duration of life of patients with MPS type II is relevant.

## Advantages of the invention

The above-mentioned problems are successfully solved in the present invention, which relates to a compound, a pharmaceutical composition, intended for the treatment of a lysosomal storage disease, containing a therapeutic enzyme and a transport element, connected to each other directly or by means of a linker, wherein said transport element is a Fab fragment of immunoglobulin IgG, specific for an epitope in the insulin receptor. The invention is based on the unexpected discovery that the transport element, which is a Fab fragment of immunoglobulin IgG, specific for an epitope in the insulin receptor, connected directly or via a linker with a therapeutic enzyme, unexpectedly leads to an improvement in the ability of the enzyme to be transported to lysosomes of various organs and tissues, including lysosomes of nervous tissue cells, while the enzymatic activity of the compound remains at a high level. Due to this unexpected effect, progress is achieved in the field of enzyme replacement therapy for lysosomal storage diseases such as mucopolysaccharidosis type II, and an increase in the duration and improvement of the quality of life of subjects suffering from the lysosomal storage disease,mucopolysaccharidosis type II, and requiring therapy.

In addition, an unexpected increase in the enzymatic activity of a compound containing a transport element, which is a Fab fragment of immunoglobulin IgG, specific for an epitope in the insulin receptor (human insulin receptor, or HIR), connected directly or via a linker to a therapeutic enzyme, was discovered, compared to a therapeutic enzyme without a transport element; and also that the administration of a compound containing a transport element, which is a Fab fragment of immunoglobulin IgG, specific for an epitope in the insulin receptor, connected directly or via a linker to a therapeutic enzyme, provides a higher degree of substitution of the activity of the therapeutic enzyme in the human brain compared to compounds containing a Mab fragment.

The HIR-Fab-IDS compound was found to penetrate into the patient's brain tissue 2 times better than the HIR-Mab-IDS compound.

The HIR-Fab-IDS compound was found to restore IDS enzymatic function in the patient's brain tissue by 98%.

The dose at which the compound is safe and effective for subjects suffering from lysosomal storage disease, mucopolysaccharidosis type II, and requiring therapy is indicated.

A more effective pharmaceutical composition and/or administration regimen for the prophylaxis or treatment of lysosomal enzyme deficiency in a subject having mucopolysaccharidosis type II has been developed.

A more effective administration regimen, taking into account increased doses, has been developed for the prophylaxis or treatment of lysosomal enzyme deficiency in a subject with human mucopolysaccharidosis type II with neurologic component. A more effective administration regimen and/or pharmaceutical composition has been developed, taking into account increased doses, for the prophylaxis or treatment of lysosomal enzyme deficiency in a subject having mucopolysaccharidosis type II with a neurological component, related to the neuropathic form in humans.

## Summary

This summary provides a brief description of the present invention for the purpose of briefly setting forth the subject matter and nature of the present invention. The summary is presented with the understanding that it should not be used to interpret or limit the scope or meaning of the claims.

The first object of the present invention is a HIR-Fab-IDS compound represented by the first amino acid sequence SEQ ID NO:2 and the second amino acid sequence SEQ ID NO:4, for the prophylaxis or treatment of lysosomal enzyme deficiency in a subject having a lysosomal storage disease that is mucopolysaccharidosis type II.

Also an object of the present invention is a HIR-Fab-IDS compound represented by the first amino acid sequence SEQ ID NO:2 and the second amino acid sequence SEQ ID NO:4, used for the prophylaxis or treatment of lysosomal enzyme deficiency in a subject having a lysosomal storage disease that is mucopolysaccharidosis type II, wherein the subject is administered at least one dose of the HIR-Fab-IDS compound from 1 to 12 mg/kg.

Another object of the invention is a HIR-Fab-IDS compound represented by the first amino acid sequence SEQ ID NO:2 and the second amino acid sequence SEQ ID NO:4, used for the prophylaxis or treatment of lysosomal enzyme deficiency in a subject having a lysosomal storage disease, wherein the subject is administered at least one dose of the HIR-Fab-IDS compound, wherein the dose is selected from the group consisting of 3 mg/kg, 6 mg/kg, 12 mg/kg of the HIR-Fab-IDS compound.

In a particular embodiment, the subject of the present invention is a HIR-Fab-IDS compound used for the prophylaxis or treatment of a lysosomal enzyme deficiency in a subject having a lysosomal storage disease, wherein the lysosomal storage disease is mucopolysaccharidosis type II or mucopolysaccharidosis type II with a neurological component, related to the neuropathic form.

In a particular embodiment, the subject of the present invention is a HIR-Fab-IDS compound used for the prophylaxis or treatment of lysosomal enzyme deficiency in a subject having a lysosomal storage disease, which is mucopolysaccharidosis type II, wherein the subject is a human.

Another object of the invention is a pharmaceutical composition for use in the prophylaxis or treatment of lysosomal enzyme deficiency in a subject having a lysosomal storage disease that is mucopolysaccharidosis type II, wherein the composition comprises a HIR-Fab-IDS compound represented by the first amino acid sequence SEQ ID NO:2 and the second amino acid sequence SEQ ID NO:4, wherein the HIR-Fab-IDS compound is administered to the subject at least in one dose selected from the group consisting of 3 mg/kg, 6 mg/kg, 12 mg/kg of the HIR-Fab-IDS compound.

In a specific embodiment, the invention relates to a pharmaceutical composition for use in the prophylaxis or treatment of lysosomal enzyme deficiency in a subject having a lysosomal storage disease that is mucopolysaccharidosis type II, wherein the composition comprises a HIR-Fab-IDS compound, wherein the pharmaceutical composition further comprises sodium chloride, sodium dihydrogen phosphate dihydrate, sodium hydroxide, polysorbate.

In a specific embodiment, the invention relates to a pharmaceutical composition for use in the prophylaxis or treatment of lysosomal enzyme deficiency in a subject having a lysosomal storage disease that is mucopolysaccharidosis type II, wherein the composition comprises a HIR-Fab-IDS compound, wherein the pharmaceutical composition further comprises a HIR-Fab-IDS compound 5.3 mg, sodium chloride in an amount of 8.0 mg, sodium dihydrogen phosphate dihydrate in an amount of 3.12 mg, sodium hydroxide to adjust the pH to pH 6.0, polysorbate 20 in an amount of 0.2 mg, water for injection up to 1.0 mL.

In a particular embodiment, the invention relates to a pharmaceutical composition for use in the prophylaxis or treatment of a lysosomal enzyme deficiency in a subject having a lysosomal storage disease that is mucopolysaccharidosis type II, wherein the composition comprises a HIR-Fab-IDS compound, wherein the lysosomal storage disease is mucopolysaccharidosis type II with a neurological component related to the neuropathic form.

In a particular embodiment, the invention relates to a pharmaceutical composition for use in the prophylaxis or treatment of a lysosomal enzyme deficiency in a subject having a lysosomal storage disease that is mucopolysaccharidosis type II, wherein the composition comprises a HIR-Fab-IDS compound, wherein the subject is a human.

Another object of the invention is a method for prohpylaxis or treatment of a lysosomal enzyme deficiency in a subject having a lysosomal storage disease that is mucopolysaccharidosis type II, comprising administering to the patient at least one dose of the HIR-Fab-IDS compound represented by the first amino acid sequence SEQ ID NO:2 and the second amino acid sequence SEQ ID NO:4, wherein the dose is selected from the group consisting of 3 mg/kg, 6 mg/kg, 12 mg/kg of the HIR-Fab-IDS compound.

In a specific embodiment, the invention relates to a method for prohpylaxis or treatment of a lysosomal enzyme deficiency in a subject having a lysosomal storage disease that is mucopolysaccharidosis type II, comprising administering to the patient at least one dose of the HIR-Fab-IDS compound represented by the first amino acid sequence SEQ ID NO:2 and the second amino acid sequence SEQ ID NO:4, wherein the HIR-Fab-IDS compound in the pharmaceutical composition is administered intravenously for 3 hours once a week.

In a specific embodiment, the invention relates to a method for prohpylaxis or treatment of a lysosomal enzyme deficiency in a subject having a lysosomal storage disease that is mucopolysaccharidosis type II, comprising administering to the patient at least one dose of the HIR-Fab-IDS compound represented by the first amino acid sequence SEQ ID NO:2 and the second amino acid sequence SEQ ID NO:4, wherein the lysosomal storage disease is mucopolysaccharidosis type II with a neurological component related to the neuropathic form.

In a particular embodiment, the invention relates to a method for prohpylaxis or treatment of a lysosomal enzyme deficiency in a subject having a lysosomal storage disease that is mucopolysaccharidosis type II, comprising administering to the patient at least one dose of the HIR-Fab-IDS compound represented by the first amino acid sequence of SEQ ID NO:2 and the second amino acid sequence of SEQ ID NO:4, wherein the subject is a human.

In other embodiments of the present invention, the approach of the present invention can also be used to treat other lysosomal storage diseases, in particular to treat other types of mucopolysaccharidosis, such as mucopolysaccharidosis type III (Sanfilippo syndrome), including mucopolysaccharidosis type IIIA, type IIIB, type IIIS and type PGO; mucopolysaccharidosis type IV (Morquio syndrome), including mucopolysaccharidosis type IVA and type IVB; mucopolysaccharidosis type VI (Maroteaux-Lamy syndrome), mucopolysaccharidosis type VII (Sly syndrome) and mucopolysaccharidosis type IX. In these cases, instead of $\alpha$-L-iduronidase or iduronate-2-sulfatase, another therapeutic enzyme is used as the corresponding therapeutic enzyme, the deficiency of which is encountered by patients with the corresponding form of mucopolysaccharidosis: heparan sulfamidase in mucopolysaccharidosis type IIIA, N-acetyl glucosaminidase in mucopolysaccharidosis type IIIB, heparan-$\alpha$-glucosaminid-N-acetyltransferase in mucopolysaccharidosis type IIIC, N-acetylglucosamine-6-sulfatase in mucopolysaccharidosis type PGO, galactose-6-sulfate sulfatase in mucopolysaccharidosis type IVA, $\beta$-galactosidase in mucopolysaccharidosis type IVB, N-acetylgalactosamine-4-sulfatase in mucopolysaccharidosis type VI, $\beta$-glucuronidase in mucopolysaccharidosis type VH and hyaluronidase for mucopolysaccharidosis type IX. The amino acid sequences of these therapeutic enzymes can be obtained using any of a number of computer programs known in the art, such as BLAST or FASTA, etc. Both BLAST and FASTA allow offline and online searching (see Ausubel et al., 1999 ibid, pages 7-58-7-60, National Center for Biotechnology Information website, National Institutes of Health website).

The approach of the present invention, in alternative embodiments, may also be used to treat lysosomal storage diseases other than mucopolysaccharidoses by using a different lysosomal enzyme in place of $\alpha$-L-iduronidase or iduronate-2-sulfatase, the deficiency of which is experienced by patients with the corresponding lysosomal storage disease. In certain non-limiting embodiments of the present invention, the approach of the present invention may be used, in particular, to treat amino acid metabolism disorders, such as cystinosis; to treat carbohydrate metabolism disorders, such as glycogen storage diseases, in particular Pompe disease; sphingolipid metabolism disorders and other lipid storage diseases, in particular, GM2 gangliosidosis, including Sandhoff disease and Tau-Sachs disease; other gangliosidoses, in particular GMi gangliosidosis and mucolipidosis IV; other sphingolipidoses, in particular Fabry disease, Gaucher disease, Krabbe disease, Niemann-Pick disease, Farber syndrome, metachromic leukodystrophy and multiple sulfatase deficiency; neuronal lipofuscinosis, in particular Batten disease, Bielschowsky-Jansky disease, Kufs disease, Spielmeyer-Vogt disease; other lipid storage disorders, in particular Wolman disease, and for the treatment of glycoprotein metabolism disorders including mucolipidosis II (I-cell disease), mucolipidosis III (Hurler pseudolipodystrophy), and for the treatment of glycoprotein degradation defects including aspartylglucosaminuria, fucosidosis, mannosidosis and sialidosis.

In these cases, the amino acid sequence of the corresponding enzyme, the deficiency of which is encountered by patients with lysosomal storage disease, is attached directly or via a linker to the transport element instead of iduronate-2-sulfatase.

The most preferred embodiments of the present invention are described below. However, these preferred embodiments are provided only for the purpose of illustrating the present invention, and not for limiting the scope of the claims.

## Brief description of the figures

The invention is illustrated by the following figures.

Figure 1 shows a representative autoradiogram of a male cynomolgus macaque recorded 2 hours after a single intravenous injection of [$^{125}$I]-HIR-Fab-IDS at a nominal dose level of 0.0020 mg/kg body weight, with the numbers indicating:

1. Cerebral cortex
2. Cerebral medulla
3. Eye
4. Hypothalamus
5. Pons
6. Cerebellum
7. Medulla oblongata
8. Spinal cord
9. Myocardium
10. Blood
11. Lung
12. Liver
13. Stomach
14. Kidney
15. Small intestine
16. Large intestine
17. Muscle
18. Testis
19. Bone marrow

Figure 2 shows a representative autoradiogram of a male cynomolgus macaque recorded 2 hours after a single intravenous injection of [$^{125}$I]-HIR-Mab-IDS at a nominal dose level of 0.0015 mg/kg body weight, with the numbers indicating:

1. Cerebral cortex
2. Cerebral medulla
3. Eye
4. Hypothalamus
5. Pons
6. Cerebellum
7. Medulla oblongata
8. Spinal cord
9. Myocardium
10. Blood
11. Lung
12. Liver
13. Stomach
20. Adrenal
14. Kidney
15. Small intestine

21. Bladder
19. Bone marrow

Figure 3 shows a representative autoradiogram of a male cynomolgus macaque recorded 2 hours after a single intravenous injection of [$^{125}$I]-IDS (control) at a nominal dose level of 0.0010 mg/kg body weight, with the numbers indicating:

1. Cerebral cortex
2. Cerebral medulla
3. Eye
4. Hypothalamus
5. Pons
6. Cerebellum
7. Medulla oblongata
8. Spinal cord
9. Myocardium
10. Blood
11. Lung
12. Liver
13. Stomach
14. Kidney
15. Small intestine
16. Large intestine
17. Muscle
18. Testis
19. Bone marrow

Fig. 4 shows the level of GAG in the urine of animals during the study. Individual values. The following are indicated in Latin letters in Fig. 4:

A. Wild-type mice
B. Knockout mice, saline solution
C. Knockout mice, 0.3 mg/kg
D. Knockout mice, 1.0 mg/kg
E. Knockout mice, 3.0 mg/kg.

Fig. 5 shows the liver weight in animals of the study groups. I - wild-type group; II - group - knockout animals of the control group; III - knockout animals, 0.3 mg/kg; IV - knockout animals, 1.0 mg/kg; V - knockout animals, 3.0 mg/kg. Individual values.

Fig. 6 shows the level of iduronate-2-sulfatase activity in the plasma of experimental animals. I - wild type group; II - group - knockout animals of the control group; III - knockout animals, 0.3 mg/kg; IV - knockout animals, 1.0 mg/kg, V - knockout animals, 3.0 mg/kg. Individual values.

Fig. 7 shows the serum concentrations of the HIR-Fab-IDS compound in patient 1001 determined during the dose escalation step. The NICO of the method is 50 ng/mL.

Fig. 8 shows the dynamics of the concentration of heparan sulfate in the CSF when using different doses of the drug.

## Detailed description

Unless otherwise defined, all technical and scientific terms used in this specification have the same meaning as commonly understood by one of ordinary skill in the art (e.g., molecular genetics, nucleic acid chemistry, protein chemistry, biochemistry, organic chemistry, immunology, microbiology, genetics, etc.).

In the context of the present description, the term "compound" is understood in the broadest sense as at least one molecule that is a conjugate, a fusion protein, a fusion antibody, a hybrid protein, a fusion protein, a protein construct, a protein complex, etc.

A compound intended for the treatment of a lysosomal storage disease, comprising a therapeutic enzyme and a transport element linked to each other directly or via a linker, wherein the transport element is a Fab fragment of immunoglobulin IgGl consisting of the light chains of immunoglobulin IgG1 to the insulin receptor (SEQ ID NO:2) and the heavy chain with idursulfase (SEQ ID NO:4)

As used herein, the term "containing" means a compound that includes the listed elements without excluding others.

In the present description, the terms "therapeutic enzyme" and "enzyme" are synonymous and refer to an enzyme for the treatment of diseases arising due to the absence, deficiency, dysfunction of the enzyme, etc., while the subject suffering from diseases can be treated by ERT, enzyme administration, etc. In particular, the enzyme can be an enzyme for treating diseases that can arise due to the absence, deficiency, and dysfunction of the lysosomal enzyme, but is not limited to it.

A therapeutic enzyme that is part of the compound of the present description encompasses any enzyme that has a therapeutic effect on a lysosomal storage disease, including, but not limited to, β-glucosidase, β-galactosidase, galactose-6-sulfatase, acid ceramidase, acid sphingomyelinase, galactocerebrosidase, arylsulfatase A, β-hexosaminidase A, β-hexosaminidase B, heparin-N-sulfatase, α-D-mannosidase, β-glucuronidase, N-acetylgalactosamine-6-sulfatase, lysosomal acid lipase, α-N-acetyl-D-glucosaminidase (NAGLU), glucocerebrosidase, butyrylcholinesterase, chitinase, glutamate decarboxylase, lipase, uricase, platelet activating factor acetylhydrolase, neutral endopeptidase, myeloperoxidase, acetyl-CoA-glucosaminid-P-acetyltransferase, N-acetylglucosamine-6-sulfatase, galactosamine-6-sulfatase (GALN), hyaluronidase, α-fucosidase, β-mannosidase, α-neuraminidase (sialidase), N-acetyl-glucosamine-1-phosphotransferase, mucolipin-1, α-N-acetyl-galactosaminidase, N-aspartyl-β-glucosaminidase, LAMP-2 (lysosome-associated membrane protein 2), cystinosin, sialin, ceramidase, acid β-glucosidase, galactosylceramidase, NPC1 (protein Niemann-Pick disease type C1), cathepsin A, SUMF-1 (sulfatase-modifying factor-1), lysosomal acid lipase (LIPA) and tripeptidyl peptidase 1.

In particular, the therapeutic enzyme includes enzymes such as agalsidase, imiglucerase, galsulfase, iduronate-2-sulfatase and α-L-iduronidase. Most preferably, the therapeutic enzyme is iduronate-2-sulfatase or α-L-iduronidase.

However, the present description may also cover any therapeutic enzyme, without limitation, regardless of the type or origin of the enzyme.

In the present description, the term **"iduronate-2-sulfatase"**, as well as **"idursulfase", "IDS", "IDS", "I2S"** means a recombinant analogue of the lysosomal enzyme iduronate-2-sulfatase, which normally hydrolyzes the O-linked sulfate group of mucopolysaccharides - dermatan and heparan sulfates. In the present invention, the term **"iduronate-2-sulfatase"** can be used interchangeably with the term **"idursulfase".**

In the context of the present description, the terms **"HIR-Fab-IDS",** as well as **"rIDS-FAB-H1", "rHIR-FAB-IDS", "rIDS-FAB-HIR"** and **"rHIR-FAB-IDS"** are synonymous and denote a recombinant iduronate-2-sulfatase fusion protein covalently linked to a Fab fragment of a monoclonal antibody to the human insulin receptor. In the context of the present description, **"HIR-Fab-IDS"** means, but is not limited to, iduronate-2-sulfatase with a fragment of a monoclonal antibody to the human insulin receptor. In particular (non-limiting) embodiments of the present invention, the HIR-Fab-IDS variants are represented by a first amino acid sequence selected from SEQ ID NO:2, 8, 9, 10 or 11, and a second amino acid sequence selected from SEQ ID NO:4, 5, 12, 13, 14 or 15. In a specific embodiment of the present invention, the HIR-Fab-IDS is represented by the first amino acid sequence of SEQ ID NO:2 and the second amino acid sequence of SEQ ID NO:4.

In the context of the present description, **"HIR-Mab-IDS"** is a recombinant iduronate-2-sulfatase fusion protein covalently linked to a full-length monoclonal antibody to the human insulin receptor. In particular (non-limiting) embodiments of the present invention, the HIR-Mab-IDS variants are those according to US patent document US8834874 B2, 16.09.2014. In a specific embodiment of the present invention, HIR-Mab-IDS is represented by the amino acid sequence of the product according to the AGT-182 project of ArmaGen Technologies Inc.

In the present description, the term **"α-L-iduronidase"**, as well as **"iduronidase", "laronidase", "IDUA"** means an enzyme involved in the hydrolysis of glycosaminoglycans such as dermatan sulfate and heparan sulfate. In the present description, the term **"α-L-iduronidase"** can be used interchangeably with the term **"laronidase".** Deficiency (genetically determined insufficiency) of iduronidase, which occurs in mucopolysaccharidosis type I, leads to a gradual accumulation of glycosaminoglycans (heparan sulfate and dermatan sulfate) in the cells and tissues of the body.

**"HIR-Fab-IDUA",** as well as **"rIDUA-FAB-HI", "rHIR-FAB-IDUA", "rIDUA-FAB-HIR", "rHIR-FAB-IDS"** is a hybrid recombinant protein α-L-iduronidase covalently linked to the Fab fragment of a monoclonal antibody to the human insulin receptor. In particular (non-limiting) embodiments of the present invention, the HIR-Fab-IDUA variants are represented by the first amino acid sequence selected from SEQ ID NO:2, 8, 9, 10 or 11, and a second amino acid sequence selected from SEQ ID NO:6, 7.

**HIR-Mab-IDUA** is a recombinant fusion protein of α-L-iduronidase covalently linked to a full-length monoclonal antibody to the human insulin receptor.

Therapeutic enzymes that can be included in the compounds of the present invention may be in their native form, as well as in the form of fragments consisting of parts of enzymes, or enzyme analogs in which a variation has arisen selected from the group consisting of substitution, addition, deletion, modification of certain amino acids and combinations thereof, without limitation, provided that they have the same enzymatic activity as the native forms of the corresponding therapeutic enzymes. In particular (non-limiting) embodiments of the invention, enzyme fragments having the activity of the native forms of the corresponding enzymes can be used. Enzyme analogs, without limitation, are those enzymes that have different glycosylation characteristics and the degree of glycosylation caused by the expression of a known enzyme in different hosts, as well as a different degree of substitution of specific amino acid residues of the corresponding enzyme relative to the standard sequence, where the degree of substitution is not a 100% replacement. In particular (non-limiting) embodiments of the invention, analogs of α-L-iduronidase known from patents US5932211 A, 03.08.1999, US6153188 A, 28.11.2006 and US6541254 B1, 01.04.2003, as well as analogs of iduronate-2-sulfatase can be used. A person skilled in the art understands that other fragments and analogs of the enzymes α-L-iduronidase and iduronate-2-sulfatase can be used, possessing the activity of the native forms of the corresponding enzymes, which are currently known from the state of the art or will become known in the future.

The enzymes can be produced by conventional techniques in the art, such as genetic recombination in animal cells, E. coli, yeast, insects, plants and living animals, etc., using various expression vectors well known to those skilled in the art. The production methods are not limited to those indicated and also include other methods for producing enzymes known to those skilled in the art. For non-limiting examples of such methods for producing enzymes in cells of various organisms, as well as non-limiting examples of expression vectors, see the guide by Sambrook et al. al., "Molecular Cloning: A Laboratory Manual", CSH Press, Cold Spring Harbor, 1989; "Current Protocols in Molecular Biology", John Wiley & Sons, New York, 2001. In preferred embodiments, the enzymes are produced in mammalian cells. In the most preferred embodiments, the enzymes are produced in Chinese hamster ovary cells.

In certain preferred embodiments of the invention, the enzymes may be commercially available enzymes. Furthermore, the enzymes may comprise an amino acid sequence that has a homology of at least 80%, more particularly 90%, and even more particularly 91%, 92%, 93%, 94%, 95%, 96%, 97%, 98% or 99% or higher with the above enzymes or their analogs, and the enzymes may be obtained from microorganisms using recombinant technology or may be purchased from commercial sources, without limitation.

In the present description, the term "homology" means the degree of similarity with an amino acid sequence of a wild-type protein or a nucleotide sequence encoding amino acids, and covers sequences that have the above-mentioned degree of sequence similarity, expressed as a percentage, with the amino acid sequences or nucleotide sequences of the present invention. Homology can be determined by comparing two given sequences with the unaided eye or can be determined using a bioinformatics algorithm that allows for homology analysis by aligning the sequences in question for comparison. Homology between two given amino acid sequences can be indicated as a percentage. There are efficient automated algorithms that can be used in the GAP, BESTFIT, FASTA and TFASTA software modules of the Wisconsin Genetics software package (Genetics Computer Group, Madison, WI, USA). The alignment algorithm automated in these modules includes the Needleman and Wunsch, Pearson and Lipman, and Smith and Waterman sequence alignment algorithms. Other algorithms that can be used for sequence alignment and homology determination are automated in the programs FASTP, BLAST, BLAST2, PSIBLAST, and CLUSTAL W. Amino acid sequences and nucleotide sequences encoding enzymes and their analogs can be taken from a well-known database such as, but not limited to, NCBI GenBank.

In some embodiments of the invention, the transport element of the compound comprises a Fab fragment of immunoglobulin IgG1 consisting of a first amino acid sequence that is at least 50, 55, 60, 65, 70, 75, 80, 81, 82, 83, 84, 85, 86, 87, 88, 89, 90, 91, 92, 93, 94, 95, 96, 97, 98, 99% or more identical to SEQ ID NO:2.

In specific embodiments of the invention, the transport element of the compound comprises a Fab fragment of immunoglobulin IgG1, consisting of a first amino acid sequence that is at least 80% or more identical to SEQ ID NO:2.

In some embodiments of the invention, a compound represented by a first amino acid sequence at least 50, 55, 60, 65, 70, 75, 80, 81, 82, 83, 84, 85, 86, 87, 88, 89, 90, 91, 92, 93, 94, 95, 96, 97, 98, 99% or more identical to SEQ ID NO:2, and a second amino acid sequence at least identical to SEQ ID NO:4, used to treat or prevent a lysosomal enzyme deficiency in a subject having mucopolysaccharidosis type II.

In specific embodiments of the invention, a compound represented by a first amino acid sequence at least 80% or more identical to SEQ ID NO:2 and a second amino acid sequence at least identical to SEQ ID NO:4 is used to treat or prevent a lysosomal enzyme deficiency in a subject having mucopolysaccharidosis type II.

The term "amino acid" refers to a group of carboxy-α-amino acids that are either naturally occurring, i.e., may be encoded directly or as a precursor by a nucleic acid, or not naturally occurring. Individual naturally occurring amino acids are encoded by nucleic acids consisting of three nucleotides, called codons or base triplets. Each amino acid is encoded by at least one codon.

The term **"amino acid"** as used herein refers to naturally occurring carboxy-α-amino acids including the following: alanine (three-letter code: Ala, one-letter code: A), arginine (Arg, R), asparagine (Asn, N), aspartic acid (Asp, D), cysteine (Cys, C), glutamine (Gin, Q), glutamic acid (Glu, E), glycine (Gly, G), histidine (His, H), isoleucine (IIe, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y) and valine (Val, V). Examples of amino acids that are not found in nature (non-proteinogenic amino acids) include Aad (alpha-aminoadipic acid), Abu (aminobutyric acid), Ach (alpha-aminocyclohexanecarboxylic acid), Acp (alpha-aminocyclopentanecarboxylic acid), Acpc (1-aminocyclopropane-1-carboxylic acid), Aib (alpha-aminoisobutyric acid), Aic (2-aminoindan-2-carboxylic acid; also called 2-2-Aic), 1-1-Aic (1-aminoindan-1-carboxylic acid), (2-aminoindan-2-carboxylic acid), allylglycine (allylglycine), alloisoleucine (allo-Ne), Asu (alpha-aminosuberic acid, 2-aminooctanedioic acid), Bip (4-phenyl- phenylalanine carboxylic acid), BnHP ((28,4K)-4-hydroxyproline), Cha (beta-cyclohexylalanine), Cit (citrulline), cyclohexylglycine (Chg), cyclopentylalanine, beta-cyclopropylalanine, Dab (1,4-diaminobutyric acid), Dap (1,3-diaminopropionic acid), p-(3,3-diphenylalanine carboxylic acid), 3,3-diphenylalanine, di-n-propylglycine (Dpg), 2-furylalanine, homocyclohexylalanine (HoCha), homocitrulline (HoCit), homocycloleucine, homoleucine (HoLeu), homoarginine (HoArg), homoserine (HoSer), hydroxyproline, Lys(Ac), (1) Nal (1-naphthylalanine), (2) Nal (2-naphthylalanine), 4-MeO-Ars (1-amino-4-(4-methoxyphenyl)-cyclohexane-1-carboxylic acid), nor-leucine (Nle), Nva (norvaline), omatin, 3-Pal (alpha-amino-3-pyridylalanine-carboxylic acid), 4-Pal (alpha-amino-4-pyridylalanine-carboxylic acid), 3,4,5,F3-Phe (3,4,5-trifluoro-phenylalanine), 2,3,4,5,6,F5-Phe (2,3,4,5,6-pentafluoro-phenylalanine), Pqa (4-oxo-6-(1-piperazinyl)-3(4H)-quinazoline-acetic acid (CAS 889958-08-1)), pyridylalanine, quinolylalanine, sarcosine (Sar), thiazolyl alanine, thienyl alanine, Tic (alpha-amino-1,2,3,4-tetrahydroisoquinoline-3-carboxylic acid), Tic(OH), Tie (tert-butylglycine), and Tyr(Me), but are not limited to.

The term **"amino acid sequence"** refers to polypeptides having amino acid sequences that differ to some extent from the native sequence polypeptide of the corresponding therapeutic enzyme. Typically, amino acid sequence variants will have at least about 70% sequence identity to the native sequence polypeptide of the corresponding therapeutic enzyme. In one embodiment, the variant has about 80% or more sequence identity to the native sequence polypeptide of the corresponding therapeutic enzyme. In one embodiment, the variant has about 90% or more sequence identity to the native sequence polypeptide of the corresponding therapeutic enzyme. In one embodiment, the variant has about 95% or more sequence identity with the native sequence polypeptide of the corresponding therapeutic enzyme. In one embodiment, the variant has about 98% or more sequence identity with the native sequence polypeptide of the corresponding therapeutic enzyme. Amino acid sequence variants have substitutions, deletions and/or insertions at certain positions within the amino acid sequence of the native amino acid sequence of the corresponding therapeutic enzyme. Amino acids are designated by traditional names, one-letter codes and three-letter codes.

The term "first amino acid sequence" as used in the present invention refers to an amino acid sequence of immunoglobulin IgG in general, to a light chain of immunoglobulin IgG in particular. In particular (non-limiting) embodiments, the first amino acid sequence is an amino acid sequence of immunoglobulin IgG1, an amino acid sequence of a light chain of immunoglobulin IgG1, a fragment of an amino acid sequence of a light chain of immunoglobulin IgG1, selected from SEQ ID NO: 2, 8, 9, 10 or 11. In a particular embodiment, the first amino acid sequence is an amino acid sequence of a light chain of immunoglobulin IgG: SEQ ID NO: 2.

The term "second amino acid sequence" as used in the present invention refers to an amino acid sequence of immunoglobulin IgG in general; to a heavy chain of immunoglobulin IgG, to a fragment of a heavy chain of immunoglobulin IgG, in particular to a fragment of a heavy chain of immunoglobulin IgG linked to an enzyme sequence directly or via a linker. In particular (non-limiting) embodiments, the second amino acid sequence is an amino acid sequence of immunoglobulin IgG1, heavy chain fragment amino acid sequence - SEQ ID NO:3, heavy chain fragment of immunoglobulin IgG1 linked to an iduronate-2-sulfatase sequence selected from SEQ ID NO:4, 5, 12, 13, 14 or 15.

The term **"transport element"** as used in this invention refers to a substance that is capable of carrying, transporting, delivering a therapeutic enzyme to the lysosomes of cells of various tissues. In particular, but not limited to, the transport element will specifically interact with an epitope, antigen, receptor or target in such a way as to ensure effective delivery to the lysosomes of cells of nervous tissue. Such an epitope, antigen, receptor or target in the context of the present description may be the human insulin receptor or a part thereof, through which the delivery of a drug, peptide or protein is carried out, including through the BBB.

**"Immunoglobulin"** is a tetrameric molecule, as used herein, a **"full-length antibody".** In naturally occurring immunoglobulin, each tetramer consists of two identical pairs of polypeptide chains, each pair having one "light" (about 25 kDa) and one "heavy" chain (about 50-70 kDa). The N-terminal portion of each chain includes a variable region of about 100 to 110 or more amino acids primarily responsible for antigen recognition. A portion of the carboxyl terminus of each chain defines a constant region primarily responsible for effector function.

In the context of the present description, the term **"antibody"** is used in its broadest sense and includes, in particular, monoclonal antibodies, polyclonal antibodies, multispecific antibodies (e.g. bispecific antibodies) formed from at least two intact antibodies, and antibody fragments, provided that they possess the required biological activity.

As used herein, **"antibody fragments"** comprise a portion of an intact antibody that retains the ability to bind to an antigen. Examples of antibody fragments include Fab, Fab', F(ab')2, and Fv fragments; dimeric antibodies (diabodies); linear antibodies; single-chain antibody molecules such as single-chain Fab, scFv, and multispecific antibodies formed from antibody fragments. Papain digestion of antibodies produces two identical antigen-binding fragments, called "Fab fragments," each with a single antigen-binding site, and a residual "Fc fragment," the name of which reflects its ability to crystallize readily.

**"Fab fragment", "Fab", "FAB"** is a monovalent fragment that contains the variable domains of the heavy and light chains and also contains the constant domain of the light chain and the first constant domain of the heavy chain. In the context of the present description, the transport element comprises the amino acid sequence Fab- an IgG immunoglobulin fragment, wherein the IgG immunoglobulin is IgG1, IgG2 or IgG4. In particular (non-limiting) embodiments of the present invention, the IgG immunoglobulin is IgG1. In a particular embodiment, the transport element comprises an amino acid sequence of an IgG1 immunoglobulin Fab fragment consisting of a first amino acid sequence selected from SEQ ID NO:2, 8, 9, 10 or 11 and a second amino acid sequence of SEQ ID NO:3. In a more particular embodiment, the transport element comprises an amino acid sequence of an IgG1 immunoglobulin Fab fragment consisting of SEQ ID NO:2 and SEQ ID NO:3.

In the context of the present description, the term "linker" refers to a chemical linker or a single-chain peptide linker that connects the therapeutic enzyme and the transport element of the compound of the present invention. The linker connects, for example, a monovalent binding element comprising the CH2-CH3 domain of Ig and an sFab targeting the insulin receptor, i.e., the linker connects the sFab to the C-terminus of the CH3-CH2-D domain of Ig.

In some embodiments, the linker is a chemical linker. Single-chain peptide linkers containing one to twenty amino acids linked by peptide bonds can be used. In specific embodiments, the amino acids are selected from twenty naturally occurring (proteinogenic) amino acids. In other specific embodiments, the one or more amino acids are selected from glycine, alanine, proline, asparagine, glutamine, and lysine. In particular (non-limiting) embodiments, the one or more amino acids are selected from glycine, serine, and leucine.

In specific embodiments of the invention, said linker is a single-chain peptide, the amino acid sequence of which consists of at least 1 amino acid, preferably 1-2 amino acids. In particular (non-limiting) embodiments of the present invention, the amino acid sequence of the linker may consist of more than 1-2 amino acids, for example, 3 or 15 amino acids.

The coupling of the therapeutic enzyme and the transport element can be accomplished directly or using a variety of chemical linkers known in the art. In certain preferred (non-limiting) embodiments of the invention, coupling of the therapeutic enzyme and the transport element can be accomplished using a variety of bifunctional protein coupling agents such as N-succinimidyl 3-(2-pyridyldithio)propionate (SPDP), succinimidyl 4-(1-maleimidomethyl)cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bisazido compounds (such as bis(p-azidobenzoyl)hexanediamine), bisdiazonium derivatives (such as bis(p-diazoniumbenzoyl)ethylenediamine), diisocyanates (such as toluene-2, 6-diisocyanate) and bis-active fluorine compounds (such as 1,5-difluoro-2,4-dinitrobenzene). One skilled in the art will also appreciate that other chemical and peptide linkers known in the art that are not explicitly described herein may be used for the purposes of the present invention.

In certain preferred embodiments of the invention, the linker may be a "cleavable linker" that facilitates the release of the therapeutic enzyme after the compound has been transported into cells and tissues. For example, an acid-labile linker, a peptidase-sensitive linker, a photolabile linker, a dimethyl linker, or a disulfide-containing linker can be used (Chari R. et al., Cancer Res. 52, 1992, pp. 127-131; US 5,208,020, Chari Ravi J. et al., 04.05.1993). One skilled in the art will also appreciate that other cleavable linkers known in the art that facilitate the release of the therapeutic enzyme after the compound has penetrated into the cells and tissues of the central nervous system can be used for the purposes of the present invention.

The term "epitope" is a region of an antigen that is bound by an antigen-binding protein, including an antibody. Epitopes can be defined as structural or functional. Functional epitopes are generally a subset of structural epitopes and have those residues that directly contribute to the affinity of the interaction. Epitopes can also be conformational, which are composed of nonlinear amino acids, in other words, conformational epitopes are composed of non-sequential amino acids. Epitopes can include determinants, which are chemically active surface groupings of molecules such as amino acids, sugar side chains, phosphoryl groups or sulfonyl groups, and may also have specific three-dimensional structural characteristics and/or specific charge characteristics. In the context of the present description, the epitope is the epitope in the insulin receptor represented by the sequence SEQ ID NO:1, described in the works of Zhang B.;, Roth R.A. (1991) Proc. Natl. Acad. Sci. USA.; 88(21): 9858-9862 and S. A. Prigent, K. K, Stanley, and K, Siddle. (1990) J. Biol. 1991.

The term "insulin receptor" (HIR) is a transmembrane glycoprotein (molecular weight of approximately 320,000 Da) composed of two $\alpha$ subunits and two $\beta$ subunits linked by disulfide bridges (the $\alpha$ subunits are located extracellularly and contain the insulin-binding domain) and is involved in the regulation of glucose absorption and distribution, as well as the synthesis and accumulation of fats, proteins, and carbohydrates in the human body. Insulin receptors and their extracellular insulin-binding domain (ECD) are well known in the art, both structurally and functionally. Insulin receptors are most commonly located on the cell surfaces of insulin-sensitive tissues, such as connective tissue cells, skeletal muscle cells, adipose tissue cells, liver cells, etc. See, for example, Yip et al. (2003), J. Biol. Chem., 278 (30): 27329-27332; and Whittaker et al. (2005), J Biol. Chem., 280 (22): 20932- 20936. In one embodiment, HIR herein is a human insulin receptor comprising the amino acid sequence set forth in Kasuya et al. (Biochemistry 32 (1993) 13531-13536).

The insulin receptor is expressed almost everywhere and the use of such a delivery route can significantly increase the bioavailability of the recombinant enzyme and increase the effectiveness of therapy. Delivery to brain tissue is carried out by transcytosis through the capillary endothelium of the central nervous system, through interaction with the human insulin receptor.

In peripheral and brain tissues (Hawkes C. et al., 2004) expressing M6PR, internalization of the chimeric molecule can be accomplished by both pathways: interaction of antibody (HIR) and enzyme (M6PR). In this case, targeted delivery to lysosomes occurs through interaction with M6P receptors. Internalization via HIR (human insulin receptor) leads to entry into early-order endosomes and subsequent transcytosis. In addition to delivery of the functional enzyme to the CNS, many lysosomal storage diseases require improved internalization by certain peripheral tissues (diaphragm muscles in Pompe disease, liver and spleen in Hunter disease, kidneys in Fabry disease, etc.).

The term **"specific"** means that the molecule referred to in the term can form a complex with a specific site on another molecule. Binding can be detected by *in vitro* assays such as plasmon resonance (BIAcore, GE-Healthcare, Uppsala, Sweden). The specific interaction of a molecule with a binding site on another molecule (affinity of complex formation) is determined by the values $k_a$ (rate constant for association of compounds to form a complex), $k_D$ (dissociation constant, dissociation of the complex) and $K_D$ ($k_D$/ka). Binding or specific binding means a binding affinity ($K_D$) of about $10^{-7}$ M or less, in one embodiment from about $10^{-8}$ M to about $10^{-13}$ M, in one embodiment from about $10^{-9}$ M to about $10^{-13}$ M.

The term **"lysosome"** refers to a cellular organelle, a type of vesicle, that contains a number of enzymes (acid hydrolases) capable of breaking down macromolecules either within the cell itself (e.g., when structural components of the cell are being processed) or taken up from outside the cell. Inherited defects or deficiencies in lysosomal enzymes (or other lysosomal components) can result in the accumulation of undegraded metabolites. Glycosaminoglycans (formerly called mucopolysaccharides) are common polysaccharides on the cell surface and in the extracellular matrix and structures. Enzyme deficiencies that prevent glycosaminoglycan degradation result in accumulation of glycosaminoglycan fragments in lysosomes and cause extensive changes in bone, soft tissue, and the central nervous system.

The **"activity"** of the enzyme(s) of the invention may be measured using any suitable test. Typically, the pH determination and the temperature determination may be adapted to the enzyme in question. Examples of pH values to be tested are pH 2, 3, 4, 5, 6, 7, 8, 9, 10, 11 or 12. Examples of temperatures to be tested are 30, 35, 37, 40, 45, 50, 55, 60, 65, 70, 80, 90 or 95°C. Preferred pH values and temperatures are in the physiological range, such as pH values of 4, 5, 6, 7 or 8 and a temperature of 30, 35, 37 or 40°C. For example, protease activity may be measured using any test in which a substrate is used that includes peptide bonds relevant to the specificity of the protease in question.

Examples of suitable enzyme tests are included in the experimental section, in particular see Example 2. As used in the present description, the term **"activity"** means **"enzyme activity", "specific activity", "enzyme specific activity"; "specific activity"** depending on the context of the description.

The term **"blood-brain barrier"** or **"BBB"** refers to the physiological barrier between the peripheral bloodstream and the brain and spinal cord that is formed by tight junctions in the endothelial plasma membranes of brain capillaries, creating a tight barrier that limits the transport of molecules into the brain, even very small molecules such as urea (60 Da). The BBB in the brain, the blood-spinal cord barrier in the spinal cord, and the blood-retinal barrier in the retina are continuous capillary barriers in the CNS and are collectively referred to herein as the blood-brain barrier (hereinafter also referred to as the BBB). The BBB also includes the blood-cerebrospinal fluid barrier.

The compounds of the present invention can be formulated into a composition, for example a pharmaceutical composition, comprising one compound or a combination of compounds or portion(s) thereof and a pharmaceutically acceptable carrier.

**"Pharmaceutical composition"** refers to a mixture of one or more of the compounds described herein, or physiologically/pharmaceutically acceptable salts or prodrugs thereof, with other chemical components such as physiologically/pharmaceutically acceptable carriers and excipients. The purpose of the pharmaceutical composition is to facilitate the administration of the compound to the body.

The pharmaceutical composition according to the present invention may include one or more pharmaceutically acceptable salts, an antioxidant, aqueous and non-aqueous carriers and/or adjuvants such as preservatives, wetting agents, emulsifiers and dispersing agents.

The term **"effective amount"** of a compound, for example in a pharmaceutical composition, refers to an amount that is effective, at dosages and for periods of time necessary, to achieve the desired therapeutic or prophylactic result (effect) in the subject undergoing treatment, with reasonable benefit/risk balance.

The term **"pharmaceutically acceptable carrier"** refers to an ingredient of a pharmaceutical composition other than the active substance (compound, agent, etc.) that is non-toxic to the subject. A pharmaceutically acceptable carrier includes, but is not limited to, a buffer, excipient, stabilizer, or preservative. In particular (non-limiting) embodiments of the present invention, a pharmaceutically acceptable carrier is co-administered with the compound.

Pharmaceutical compositions which contain the compounds used according to the present invention are prepared for storage by mixing with optional pharmaceutically acceptable carriers, excipients or stabilizers (Remington's Pharmaceutical Sciences, 16th edition, edited by Osol A., 1980), preferably in the form of lyophilized compositions or aqueous solutions. Acceptable carriers, excipients or stabilizers are non-toxic to subjects at the dosages and concentrations used and include buffers such as phosphate, citrate and other organic acid buffers; antioxidants including ascorbic acid and methionine; preservatives (such as octadecyldimethylbenzyl ammonium chloride; hexamethonium chloride;

benzalkonium chloride; benzethonium chloride; phenol; butyl or benzyl alcohol; alkyl parabens such as methyl or propyl paraben; catechol; resorcinol; cyclohexanol; 3-pentanol and meta-cresol); low molecular weight polypeptides (containing less than about 10 residues); proteins such as serum albumin, gelatin or immunoglobulins; hydrophilic polymers such as poly(vinylpyrrolidone); amino acids such as glycine, glutamine, asparagine, histidine, arginine or lysine; monosaccharides, disaccharides and other carbohydrates including glucose, mannose or dextrins; chelating agents such as EDTA; sugars such as sucrose, mannitol, trehalose, or sorbitol; salt-forming counterions such as sodium; metal-containing complexes (e.g., Zn-protein complexes); and/or nonionic surfactants such as TWEEN™, PLURONICS™, or polyethyleneglycol (PEG).

Preferably, the pharmaceutical composition additionally contains sodium chloride, sodium dihydrogen phosphate dihydrate, sodium hydroxide, polysorbate.

In some embodiments of the invention, the pharmaceutical composition additionally contains the HIR-Fab-IDS compound 5.3 mg, sodium chloride in an amount of 8.0 mg, sodium dihydrogen phosphate dihydrate in the amount of 3.12 mg, sodium hydroxide to correct pH to pH 6.0, polysorbate 20 in the amount of 0.2 mg, water for injection up to 1.0 mL.

The pharmaceutical compositions used in the present description may, if necessary, also contain more than one active substance (drug) active against a lysosomal storage disease, optionally substances with additional activities that do not adversely affect each other. The type and effective amounts of such drugs depend, for example, on the amount of the compound containing the therapeutic enzyme and the transport element present in the composition and the clinical parameters of the subjects.

The pharmaceutical compositions provided herein may be administered to a patient by any suitable route, such as intravenously by bolus injection or continuous infusion over a period of time, intramuscularly, intraperitoneally, intracerebrospinal, transdermally, subcutaneously, intra-articularly, sublingually, intrasynovially, orally, by inhalation, topically or externally. Intravenous administration is preferred.

In the context of the present description, the term "dose" refers, for example, to the dose used when the pharmaceutical composition provided herein is administered to a patient for the first time "initial dose", or when the pharmaceutical composition provided herein is administered as a dose for continuous administration "therapeutic dose".

In general, single doses in the range of about 0.3 to 30 mg/kg of the patient's body weight are considered, more often in the range of 1 to 12 mg/kg.

Typically, treatment is started with low doses of the HIR-Fab-IDS compound. For example, the initial dose of antibodies is administered to the patient, for example, by injection or infusion. The initial dose should contain about 3 to 12 mg/kg.

The initial dose is in the range of 3 to 12 mg/kg and is, for example, about 3 mg/kg, about 3.5 mg/kg, about 4 mg/kg, about 4.5 mg/kg, about 5 mg/kg, about 5.5 mg/kg, about 6 mg/kg, about 6.5 mg/kg, about 7 mg/kg, about 7.5 mg/kg, about 8 mg/kg, about 8.5 mg/kg, about 9 mg/kg, about 9.5 mg/kg, about 10 mg/kg, about 10.5 mg/kg, about 11 mg/kg, about 11.5 mg/kg, about 12 mg/kg.

Since the dose for continuous administration (therapeutic) is approximately equal to the initial dose or less or more, the dose can be varied within the limits specified and several doses during the continuous administration period do not necessarily have to be the same dose. For example, the dose can be gradually reduced or different doses can be arbitrarily repeated.

The therapeutic dose is in the range from 1 to 12 mg/kg and is, for example, about 1 mg/kg, about 1.5 mg/kg, about 2 mg/kg, about 2.5 mg/kg, about 3 mg/kg, about 3.5 mg/kg, about 4 mg/kg, about 4.5 mg/kg, about 5 mg/kg, about 5.5 mg/kg, about 6 mg/kg, about 6.5 mg/kg, about 7 mg/kg, about 7.5 mg/kg, about 8 mg/kg, about 8.5 mg/kg, about 9 mg/kg, about 9.5 mg/kg, about 10 mg/kg, about 10.5 mg/kg, about 11 mg/kg, about 11.5 mg/kg, about 12 mg/kg.

In different cases, other treatment regimens may be required, for example, a therapeutic dose may correspond to 3 mg/kg, a therapeutic dose may correspond to 6 mg/kg, a therapeutic dose may correspond to 12 mg/kg, etc.

More preferably, the therapeutic dose is selected from the group consisting of 3 mg/kg, 6 mg/kg, 12 mg/kg of the HIR-Fab-IDS compound.

In addition to expressing the dose value in mg/kg, the dose value may be expressed as a fixed dose (mg/body) and/or as a dose per unit body surface area (mg/m$^2$) corresponding to the dose per unit body mass specified above.

There is no specific limitation on the number of doses to be administered continuously, such as once, twice, three times, four times, five times, six times, seven times, eight times, nine times, ten times, 15 times, 20 times, 25 times, 35 times, 40 times, 50 times, 60 times, 70 times, 80 times, 90 times, 100 times, 500 times, 1000 times and 10000 times.

**"Interval between administrations"** (interval between individual administrations) is the interval between the administration of the initial dose and the administration of the first dose for continuous administration and the interval between the administration of the nth dose for continuous administration (n is an integer of 1 or higher) and the administration of the (n+1)-th dose for continuous administration. The interval between administrations may be one or more days, for example, 2 days, 3 days, 4 days, 5 days, 6 days, 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 1 month, 2 months, 3 months, 4 months, 5 months, 6 months, 7 months, 8 months, 9 months, 10 months, 11 months or 1 year. The interval between administrations can also be expressed in other terms, such as once a day, once every 2 days, once every 3 days, once every 4 days, once every 5 days, once every 6 days, once a week, once every 2 weeks, once every 3 weeks, once every 4 weeks, once every 5 weeks, once every 6 weeks, once every 7 weeks, once every 8 weeks, once every 9 weeks, once every 10 weeks, once every 11 weeks, once every 12 weeks, once every 13 weeks, once every 14 weeks, once every 15 weeks, once every 16 weeks, once every 17 weeks, once every 18 weeks, once every 19 weeks, once every 20 weeks, once every 21 weeks, once every 22 weeks, once every 23 weeks, once every 24 weeks, once every 25 weeks, once a month, once every 2 months, once every 3 months, once every 4 months, once every 5 months, once every 6 months, once every 7 months, once every 8 months, once every 9 months, once every 10 months, once every 11 months, or once a year. In addition, the interval between administrations can be expressed in other terms such as every day, every 2 days, every 3 days, every 4 days, every 5 days, every 6 days, every week, every 2 weeks, every 3 weeks, every 4 weeks, every 5 weeks, every 6 weeks, every 7 weeks, every 8 weeks, every 9 weeks, every 10 weeks, every 11 weeks, every 12 weeks, every 13 weeks, every 14 weeks, every 15 weeks, every 16 weeks, every 17 weeks, every 18 weeks, every 19 weeks, every 20 weeks, every 21 weeks, every 22 weeks, every 23 weeks, every 24 weeks, every 25 weeks, every month, every 2 months, every 3 months, every 4 months, every 5 months, every 6 months, every 7 months, every 8 months, every 9 months, every 10 months, every 11 months, or every year.

The interval between the initial dose and the first continuous dose and the interval between the nth continuous dose (n being an integer of 1 or greater) and the (n+1)th continuous dose may be the same; however, the intervals do not have to be the same. For example, the interval between the initial dose and the first continuous dose may be longer than the interval between the nth continuous dose and the (n+1)th continuous dose; or the interval between the initial dose and the first continuous dose may be shorter than the interval between the nth continuous dose. and the administration of the (n+1)th dose for continuous administration. In addition, the interval between the administration of the nth dose for continuous administration and the administration of the (n+1)th dose for continuous administration and the interval between the administration of the mth dose for continuous administration (m denotes an integer of 1 or higher) and the administration of the (m+1)th dose for continuous administration may be the same; however, these intervals do not necessarily have to be the same or different. For example, as the number of times of administration of the dose for continuous administration increases, the interval between administrations may be lengthened or shortened.

More preferably, the interval between administrations was one week (7 days).

The interval between dose administrations is also understood as the interval between administrations of pharmaceutical compositions.

The administration schedule is determined, for example, based on effects and safety considerations. In addition, the administration schedule is determined based on patient convenience, in a range that does not impair efficacy and safety.

In the context of the present invention, the term "almost the same" means that the difference is about 20% or less, preferably the difference is 10% or less, or more preferably the difference is 5% or less, 4% or less, 3% or less, 2% or less, or 1% or less.

The administration of doses of the claimed therapeutic compound can be carried out by any suitable route well known to a person skilled in the art, for example by injections such as intravenous, intramuscular or subcutaneous injections. The choice of a specific route of administration of the therapeutic compound is made by a person skilled in the art and depends, in particular, on whether the administration is short-term or long-term. It will be understood by a person skilled in the art that other routes of administration known in the art may also be used to administer doses of the claimed therapeutic compound, such as (without limitation) intrathecal administration, intraarterial administration, intraperitoneal administration, transdermal administration, inhalation administration, buccal administration, intranasal administration, oral administration, sublingual administration or transnasal administration (Felice B.R., Wright T.L., Boyd R.B. Safety Evaluation of Chronic Intrathecal Administration of Idursulfase-GG in Cynomolgus Monkeys. - Toxicol. Pathol. 2011 Aug.; 39(5):879-9, WO 2011/044542 A1).

In the context of the present description, various dosage regimens may be contemplated, including, but not limited to, single or multiple administrations at different time points, bolus administration, and pulsatile infusion.

For treatment, the effective dose of the HIR-Fab-IDS compound ranges from 1 mg/kg body weight to 12 mg/kg body weight, preferably from about 3 mg/kg body weight to 12 mg/kg body weight, administered weekly (once a week), preferably by intravenous administration, preferably over 3 hours.

Each vial of the drug is intended for single use only and contains 15 mg of the HIR-Fab-IDS compound in 3 mL of solution. Before intravenous administration, the drug concentrate must be diluted with 0.9% sodium chloride solution.

The terms **"approximately"** and **"about"** denote all values that are expressed as numbers with one or two decimal places.

**"Subject", "individual"** , or **"patient"** is a mammal. Mammals include, but are not limited to, domestic animals (e.g., cows, sheep, cats, dogs, and horses), primates (e.g., humans and non-human primates such as monkeys, particularly apes), rabbits, and rodents (e.g., mice and rats). In some embodiments, the subject or patient is a human.

In the context of the present description, the term **"treatment"** (and its grammatical variations such as "treat" or "treatment process") refers to a clinical intervention with the purpose of changing the natural course of a disease in an individual to be treated, and can be carried out either prophylactically or during the development of a clinical pathology. Desirable treatment actions include (but are not limited to) preventing the onset or recurrence of a disease, alleviating symptoms, reducing any direct or indirect pathological consequences of the disease, preventing metastasis, slowing the rate of disease progression, alleviating or temporarily weakening the disease state, and remission or improving the prognosis. In some embodiments, the present compounds are used to delay the development of a disease or slow the progression of a disease.

In the context of the present description, the term **"prophylaxis"** or "prevention" includes prophylactic administration and may reduce the incidence or likelihood of occurrence of a disease (e.g., MPS type II with a neurological component).

Delivery or transport of the HIR-Fab-IDS compound to a subject (e.g., by direct administration) and the practice of other methods as described herein can be used to reduce, treat, prevent, or otherwise ameliorate any suitable aspect of the progression of a lysosomal storage disease (namely, the progression of MPS II with neurological component). Methods that reduce, prevent, or otherwise lessen the intensity of such aspects of the progression of MPS II with a neurological component, individually or collectively, represent advantageous features.

In another aspect, a method is provided for reducing the risk of progression of MPS type II with a neurological component, reducing the risk of further progression of MPS type II with a neurological component that has been initiated, and/or providing a treatment regimen for reducing the progression of MPS type II with a neurological component in a human, which comprises administering to the patient one or more first-line therapies in an amount and according to a regimen sufficient to achieve a response (partial or complete response), and then administering an amount of a HIR-Fab-IDS compound to the patient or comprises administering the compound to the patient as a first-line therapy.

In a further aspect, a method is provided for inducing remission of MPS type II with a neurological component in a subject, for example a human, comprising administering a composition containing HIR-Fab-IDS compounds to the subject in order to induce remission of MPS type II with a neurological component in the subject.

In yet another additional aspect, a method is proposed for reducing the risk of developing MPS type II with a neurological component, reducing the time to MPS type II with a neurological component diagnosed at early stages, comprising administering to a patient a prophylactically effective amount of the HIR-Fab-IDS compound.

In a further aspect, a method is provided for increasing the probability of survival during a relevant period in a person diagnosed with MPS type II. In another aspect, a method is provided for improving the quality of life of a subject with MPS type II, comprising administering to the patient a composition in an amount effective to improve the patient's quality of life.

The term **"CNS"** or **"central nervous system"** refers to the complex of nerve tissues that control body function and includes the brain and spinal cord.

As used herein, the term **"lysosomal storage disease"** (LSD) refers to a rare genetic disorder that results in the loss of lysosomal functions described above due to partial or complete loss of activity of one of the lysosomal enzymes. In this case, ERT is required to replenish the enzyme whose activity is completely or partially lost or the missing enzyme. In the present description, the term **"lysosomal storage disease"** is used interchangeably with the term **"lysosomal storage disorder"** (may also be referred to as **"lysosome storage disorder").** Lysosomal storage diseases can be classified depending on the enzyme defect or deficiency as: (i) sphingolipidosis, (ii) mucopolysaccharidosis, (iii) glycogen storage disease, (iv) mucolipidosis, (y) oligosaccharidosis, (vi) lipidosis, (vii) lysosomal transport disorder, etc.

Below, lysosomal storage diseases will be described in more detail according to their classification.

In the present description, the term **"sphingolipidosis"** refers to a genetically determined deficiency syndrome of a lysosomal enzyme that hydrolyzes the carbohydrate side chains or choline side chains of sphingolipids. The diseases are classified according to the distribution of each stored lipid, such as Krabbe disease caused by galactocerebrosidase deficiency, Fabry disease caused by $\alpha$-galactosidase A deficiency, Niemann-Pick disease caused by sphingomyelinase deficiency, Gaucher disease caused by glucocerebrosidase deficiency, Tau-Sachs disease caused by hexosaminidase A deficiency, and so on, and they are diseases that are inherited in an autosomal recessive manner, except for Fabry disease, which is an X-linked genetic disease.

In the present description, the term **"mucopolysaccharidosis"** (MPS) refers to a syndrome with genetic deficiency of mucopolysaccharide hydrolase caused by deficiency of carbohydrate chain degrading enzyme, sulfatase, acetyltransferase and so on. The main symptom of mucopolysaccharidosis (MPS) is excessive secretion of mucopolysaccharides in urine. Currently, MPS is classified into 6 types, among which type I disease includes Hurler syndrome and Scheie syndrome, type II includes Hunter syndrome, type III includes Sanfilippo syndrome types A, B, C and D; type IV includes Morquio syndrome A and B; type VI includes Maroteaux-Lamy syndrome and type VII includes Sly syndrome.

As used herein, the term **"glycogen storage disease"** (also known as **"glycogenosis")** refers to a disorder of inborn error of carbohydrate metabolism caused by glycogen storage and is divided into subtypes I-VII. The subtypes of glycogen storage disease associated with lysosomal storage disease (LSD) are types II (Pompe disease) and IIIb (Danon disease).

A detailed description of the lysosomal storage diseases described herein and those disclosed in Table 1 is provided in: The Online Metabolic & Molecular Bases of Inherited Diseases (Scriver's OMMBID), Part 16 (David L. Valle, Stylianos Antonarakis, Andrea Ballabio, Arthur L. Beaudet, Grant A. Mitchell, McGraw-Hill Education, 2007).

**"Mucopolysaccharidosis type I (MPS I)"** is an inherited metabolic disorder caused by a defect in the enzyme $\alpha$-L-iduronidase (IDUA), whose function is to break down the acidic mucopolysaccharides heparan sulfate and dermatan sulfate. Insufficient levels of IDUA lead to abnormal accumulation of these mucopolysaccharides in the patient's tissues and organs, such as the heart, liver, and central nervous system. Symptoms, including neurodegeneration and mental retardation, appear in childhood and early death may occur due to organ damage. Genetic deficiency of the carbohydrate-breaking, lysosomal enzyme $\alpha$-L-iduronidase causes the lysosomal storage disease known as mucopolysaccharidosis type I (MPS I). mucopolysaccharidosis type I (MPS I) in its severe form is commonly known as Hurler syndrome and is associated with a variety of problems including mental retardation, corneal clouding, coarse facial features, heart disease, respiratory problems, enlarged liver and spleen, hernias, and joint stiffness. Patients suffering from Hurler syndrome usually die before the age of 10. In the moderate form, known as Hurler-Scheie syndrome, mental function is usually not severely affected, but physical problems can lead to death in the teens or early twenties. Scheie syndrome is a mild form of MPS I. It is compatible with a normal life span, but joint stiffness, corneal clouding, and heart valve disease cause serious problems.

**"Mucopolysaccharidosis type II (MPS II)"** or "Hunter syndrome" is an X-linked inherited metabolic disorder caused by deficiency of the enzyme iduronate-2-sulfatase (I2S). I2S is localized in lysosomes and plays an important role in the catabolism of glycosaminoglycans (GAGs) of heparan- and dermatan sulfate. In the absence of the enzyme, these substrates accumulate in cells, eventually causing congestion and then cell death and tissue destruction. Due to widespread expression of the enzyme, multiple cell types, organs, and systems are affected in patients with MPS II. The characteristic clinical feature of this disease is degeneration of the central nervous system (CNS), which leads to cognitive impairment (e.g., decreased IQ). In addition, MRI scans of patients have revealed white matter lesions, widening of perivascular spaces in the brain parenchyma, ganglia, corpus callosum, and brainstem, atrophy, and ventriculomegaly (Wang et al. Molecular Genetics and Metabolism, 2009). The disease typically presents in the first years of life with organomegaly and skeletal abnormalities. Some patients experience progressive loss of cognitive function, with most patients dying from disease-related complications in the first or second decade of life (Raluy-Callado M. et al. Orphanet. J. Rare Dis. 2013; 8: 101;).

As used herein, the term **"neurological component"** refers to a disease or disorder that affects the CNS and/or the etiology of which is related to the CNS. Examples of CNS diseases or disorders include, but are not limited to, neuropathy, amyloidosis, cancer, ocular disease or disorder, viral or microbial infection, inflammation, ischemia, neurodegenerative disease, seizure disorder, behavioral disorders, and lysosomal storage disease. Specific examples of neurological disorders include, but are not limited to, neurodegenerative diseases (including, but not limited to, Lewy body disease, postmyelitic syndrome, Shy-Drager syndrome, olivopontocerebellar atrophy, Parkinson's disease, multiple system atrophy, striatonigral degeneration), tauopathies (including, but not limited to, Alzheimer's disease and supranuclear palsy), prion diseases (including, but not limited to, bovine spongiform encephalopathy, scrapie, Creutzfeldt-Jakob syndrome, kuru, Gerstmann-Straussler-Scheinker disease, chronic wasting disease, and fatal familial insomnia), boulevard palsy, motor neuron disease, and heterodegenerative disorders of the nervous system (including, but not limited to, only them, Canavan disease, Huntington's disease, neuronal ceroid lipofuscinosis, Alexander disease, Tourette syndrome, Menkes kinky hair syndrome, syndrome Cockayne syndrome, Hallervorden-Spatz syndrome, Lafora disease, Rett syndrome, hepatolenticular degeneration, Lesch-Nyhan syndrome, and Unverricht-Lundborg syndrome), dementia (including, but not limited to, Pick's disease and spinocerebellar ataxia), cancer (e.g., cancer of the central nervous system and/or brain, including brain metastases from cancer elsewhere in the body).

In the context of this description, the term "neurological component" means "neuronopathic (with neurocognitive impairment)", "non-neuronopathic (without neurocognitive impairment)", "neurocognitive effects", "neuropathic diseases", "neuronopathic form" and any other disorders, including peripheral ones, that reflect the essence of therapy with drug penetration into/through the CNS.

MPS disorders are associated with a wide range of neurocognitive effects, ranging from mild problems with attention and executive functions to progressive and degenerative neuropathic diseases.

The neuronopathic form of MPS II presents a challenge due to the variability of the disease course, with differences in the timing of slowing and decline. MPS II has the greatest uncertainty regarding the neurocognitive progression of the disease. It is divided into two forms: a severe neuronopathic form with a major impact on neurocognitive function and a mild form that is thought to have little or no neurocognitive sequelae (Elsa G. Shapiro and Julie B. Eisengart, "The natural history of neurocognition in MPS disorders: a review". Molecular Genetics and Metabolism 133.1 (2021): 8-34).

In addition to neurocognitive impairment, central nervous system problems in MPS II include behavioral disturbances, communicating hydrocephalus, seizures, sleep apnea, and spinal cord compression. The higher risk of

# EP 4 671 275 A1

hydrocephalus and seizures in later stages of the disease may worsen the deterioration of neurocognitive functions (S. Al Sawaf, E. Mayatepek, B. Hoffmann, Neurological findings in Hunter disease:pathology and possible therapeutic effects reviewed, J. Inherit. Metab. Dis. 31(4) (2008) 473-480; I. V. Schwartz, et al., A clinical study of 77 patients with mucopolysaccharidosis type II, Acta Paediatr. 96 (455) (2007) 63-70).

The compounds proposed in the invention can be used in therapy either individually or in combination with other agents. For example, the proposed compound, containing a therapeutic enzyme and a transport element, connected linker, can be administered together with at least one additional therapeutic agent. In particular embodiments of the invention, the additional therapeutic agent is a therapeutic agent effective in treating the same neurological disorder for which the compound of the invention is used or another neurological disorder. Examples of additional therapeutic agents include, but are not limited to, the various neurological drugs described above, including cholinesterase inhibitors (such as donepezil, galantamine, rovastigmine, and tacrine), NMDA receptor antagonists (such as memantine), amyloid beta peptide aggregation inhibitors, antioxidants, $\gamma$-secretase modulators, nerve growth factor (NGF) mimics or NGF gene therapy agents, PPAR$\gamma$ agonists, HMS-CoA reductase inhibitors (statins), ampakines, calcium channel blockers, GABA receptor antagonists, glycogen synthase kinase inhibitors, intravenous immunoglobulin, muscarinic receptor agonists, nicotinic receptor modulators, active or passive immunization agents against amyloid beta peptide, phosphodiesterases, serotonin receptor antagonists and antibodies to amyloid beta peptide. Such combination therapies as mentioned above include co-administration (when two or more therapeutic agents are included in the same or in different compositions) and separate administration, in which case the administration of the compound comprising a therapeutic enzyme and a transport element connected by a linker according to the invention can be carried out before, simultaneously and/or after the administration of an additional therapeutic agent and/or adjuvant.

Some of the terms defined above may appear more than once, in which case each term must be defined independently of the other.

The following will describe in detail the exemplary embodiments of the present invention. However, each of the explanations and embodiments given as an example in this document may also be true for other explanations and exemplary embodiments. Thus, all combinations of the various factors described in this document are included in the scope of the present invention. Moreover, the scope of the present invention is not limited to the specific description given below.

It is clear to a person skilled in the art that without the expenditure of inventive creativity, additions and changes can be developed relative to what is disclosed in the examples presented below within the framework of the present invention.

## Examples

The present invention will now be described in more detail with reference to the following Examples. However, the Examples described below are provided for illustrative purposes only and are not intended to limit the invention.

## Example 1. Preparation

To prepare the compounds, animal cells were cultured and purified, into which an expression vector for animal cells was added.

Methods for producing human antibodies are currently known. For example, a human antibody of interest can be produced by immunizing a transgenic animal carrying the full spectrum of human antibody genes with the antigen of interest (see international patent application publications WO 93/12227, WO 92/03918, WO 94/02602, WO 94/25585, WO 96/34096 and WO 96/33735).

Genetically modified antibodies can also be produced by known methods. A specific example is a chimeric antibody, which is an antibody that contains the variable regions of the H chain and L chain of an antibody of an immunized animal and the constant regions of the H chain and L chain of a human antibody. Chimeric antibodies can be produced by linking DNA encoding the variable regions of an antibody derived from an immunized animal with DNA encoding the constant regions of a human antibody, inserting them into an expression vector, and then adding it into host cells to produce antibodies.

A humanized antibody is a modified antibody, which is also often referred to as a reshaped human antibody. A humanized antibody is constructed by transferring the CDRs of an antibody derived from an immunized animal into the hypervariable regions of a human antibody. General genetic recombination techniques for producing such antibodies are also known (see published European patent application EP 239400; published international patent application WO 96/02576; Sato K. et al., Cancer Research, 53 (1993), pp. 851-856; international patent application publication WO 99/51743).

*Construction of expression vectors*

The amino acid sequences of the first LC-HIR-Fab-IDS (SEQ ID NO:2) and the second HC-HIR-Fab-IDS (SEQ ID NO:4) were converted into nucleotide sequences to obtain the HIR-Fab-IDS variants, including the MDWTWRVFCLLAVAPGAHS signal peptide. For gene synthesis with subsequent cloning into pCLN-1 vectors, the following were added to the 5'-end of the sequences: the HindIII restriction site and the Kozak sequence. Two stop codons and an XbaI restriction site were added to the 3'-end of the sequences.

Optimization of the nucleotide sequence for the codon composition for Chinese hamster cells was performed using the resources: http://gcua.schoedl.de and http://www.kazusa.or.jp. Synthesis of the light and heavy chains of LC-HIR-Fab-IDS and HC-HIR-Fab-IDS was carried out at GenArt (USA) and transferred as part of the vectors pRA1675 and pRA1673 (containing the genes LC-HIR-Fab-IDS and HC-HIR-Fab-IDS, respectively).

The HC-HIR-Fab-IDS and LC-HIR-MAB sequences from pRA1675 and pRA1673 plasmids were cloned into the pCLN-1 expression vector at the HindIII/XbaI sites to yield the pGNR-055-007 (pCLN-1- HC-HIR-Fab-IDS) and pGNR-055-008 (pCLN-1- LC-HIR-Fab-IDS) vectors. The resulting vectors were linearized at the BspHI/PvuI sites.

The vectors of the present invention were constructed according to molecular biology techniques well known in the art. See Brown T, "Gene Cloning" (Chapman & Hall, London, GB, 1995); Watson R, et al., "Recombinant DNA", 2nd Ed. (Scientific American Books, New York, NY, US, 1992); Alberts B, et al., "Molecular Biology of the Cell" (Garland Publishing Inc., New York, NY, US, 2008); Innis M, et al., Eds., "PCR Protocols. A Guide to Methods and Applications" (Academic Press Inc., San Diego, CA, US, 1990); Erlich H, Ed., "PCR Technology. Principles and Applications for DNA Amplification" (Stockton Press, New York, NY, US, 1989); Sambrook J, et al., "Molecular Cloning. A Laboratory Manual" (Cold Spring Harbor Laboratory Press, Cold Spring Harbor, NY, US, 1989); Bishop T, et al., "Nucleic Acid and Protein Sequence. A Practical Approach" (IRL Press, Oxford, GB, 1987); Reznikoff W, Ed., "Maximizing Gene Expression" (Butterworths Publishers, Stoneham, MA, US, 1987); Davis L, et al., "Basic Methods in Molecular Biology" (Elsevier Science Publishing Co., New York, NY, US, 1986), Schleef M, Ed., "Plasmid for Therapy and Vaccination" (Wiley-VCH Verlag GmbH, Weinheim, DE, 2001).

*Obtaining a monoclonal cell line*

Parental cell line CHO-S. Cells were cultured at 37°C, 5% CO2, 70% humidity, in BalanCD CHO Growth A medium (Invitrogen). Stable transfection was performed on a NEON device (Invitrogen) according to the standard protocol for CHO cells using two linearized plasmids pCLN-1-HC-HIR-Fab-IDS and pCLN-1- LC- HIR-FAB-IDS. The DNA ratio for transfection was equimolar.

After 48 h, the transfected pools were plated into minipools in 96-well plates in BalanCD CHO Growth A medium containing 600 $\mu$g/mL of the selective antibiotic neomycin. The minipools were cultured under stationary conditions for 10 days at 37°C, 5% CO2, 70% humidity, after which a series of productivity screenings were performed using ELISA. The leader minipool was cloned into semi-solid ClonaCell Flex medium (STEMCEEE) for the growth of individual colonies using 6-well plates. The plates were incubated for 10 days at 37°C, 5% CO2, 70% humidity.

During screening, the concentration of the target protein HIR-Fab-IDS secreted into the culture fluid was determined. The determination was carried out by the sandwich ELISA method, using a fragment of the recombinant human insulin receptor (5 $\mu$g/mL in carbonate-bicarbonate buffer pH 9.6) instead of primary antibodies; rat antibodies to iduronate-2-sulfatase conjugated with horseradish peroxidase (diluted 1:10,000) were used as secondary antibodies. Developed with tetramethylbenzidine solution, the reaction was stopped with 0.5 M sulfuric acid.

Based on the results of screening of 6 well plates, 20 leader minipools with productivity from 0.4 to 2.1 mg/l were selected, which were transferred to flasks for adaptation to suspension cultivation. For adaptation to suspension cultivation, 3 passages were carried out, after which 20 minipools were frozen.

The selection of clones was carried out in automatic mode using the ClonePix robot (Molecular Devices) in 96-well plates. The resulting clones were screened. For this purpose, a 7-day cultivation process was simulated in the batch mode, in which the clones were assessed by the following parameters: culture viability dynamics, viable cell density dynamics, target HIR-Fab-IDS concentration, volumetric production dependence on cumulative cell density.

*Cultivation of producer cells*

The producer cells expressing HIR-Fab-IDS were cultured in a periodic fed-batch mode using the BalanCD CHO Growth A nutrient medium (Irvine Scientific) and the BalanCD CHO Feed 2 nutrient supplement (Irvine Scientific) for 12 days in a bioreactor with an overhead stirrer at a temperature of 37°C and a pH of 6.9. After the cultivation process, the culture liquid was clarified by deep filtration and transferred for isolation.

*Selection and purification*

The isolation and purification are carried out using standard protein isolation and purification techniques. The compound can be isolated or purified by any method known in the art for protein isolation or purification, such as chromatography (e.g., ion exchange, high performance liquid chromatography, affinity, protein A, and size-sorting column chromatography), centrifugation, differential solubility, or any other standard protein isolation or purification technique.

**Example 2. Determination of the level of enzymatic activity of the Fab fragment of an antibody to the insulin receptor, linked to the amino acid sequence of iduronate-2-sulfatase**

The specific activity of HIR-Fab-IDS and HIR-MAB-IDS was determined by a fluorimetric method using 4-mutilumbelliferyl-L-iduronide-2-sulfate (4-MUS) (Moscerdam Substrates, The Netherlands) (Voznyi Y.V. et al., 2001; Tolun A.A. et al., 2012; Johnson B.A. et al., 2013; Azadeh M. et al., 2017). During the assay, the substrate is hydrolyzed by iduronate-2-sulfatase to produce 4-mutilumbelliferyl-L-iduronide (MUBI), which is hydrolyzed by iduronidase (IDUA, Aldurazyme, Genzyme, USA) to 4-methylumbelliferyl (4-MU), which is detected fluorimetrically using the following fluorimeter settings: fluorescence excitation at a wavelength of 450 nm and fluorescence detection at a wavelength of 365 nm. The calibration curve is plotted using a standard 4-MU solution (Sigma-Aldrich, USA). During the assay, the mixture is first incubated at 37°C, pH 4.5 for 4 hours, then 12 $\mu$g of IDS is added and the mixture is further incubated at 37°C for 24 hours. The incubation is stopped by adding 0.2 mL of 0.5 M sodium carbonate, pH 10.3. HIR-FAB-IDS exhibits specific enzymatic (iduronate-2- sulfatase) activity towards the substrate 4-MU-$\alpha$IdoA-2S (4-methylumbelliferyl $\alpha$-L-idopyranosiduronate-2-sulfate).

*Instruments and equipment*

1. Thermostatic shaker PST-60 HL-4 (BioSan, Latvia).
2. Multifunctional reader Spectra Max M3 (Molecular Devices, USA).
3. Software for the multifunctional reader Soft Max Pro (Molecular Devices, USA).
4. Analytical scales ML 204 (Mettler Toledo, Switzerland).
5. 96-well black plate with a non-sorbent surface (Corning, USA, cat. no. 3916).

*Reagents*

1. 4-methylumbelliferone sodium salt (Sigma-Aldrich, cat. No. M1508).
2. Recombinant human $\alpha$-L-iduronidase (R&D SYSTEM, cat. no. 4119-GH).
3. 4-methylumbelliferyl $\alpha$-L-idopyranosiduronic acid 2-sulfate sodium salt, 0.5 mg/vial (USBiological, cat. no. 017551).
4. Sodium acetate anhydrous (AppliChem Panreac, cat. no. 141633.1211).
5. Acetic acid (Fluka, cat. no. 49199).
6. Bovine serum albumin (Sigma, cat. No. A7030).
7. Sodium carbonate (Sigma-Aldrich, Cat. No. S7795).
8. Sodium bicarbonate (Sigma-Aldrich, Cat. No. S6297).
9. Lead diacetate trihydrate (Aldrich, cat. no. 467863).
10. Sodium phosphate dihydrate (Sigma-Aldrich, cat. no. 71643).
11. Anhydrous citric acid (AppliChem Panreac, cat. no. 141808).

*Preparation of solutions*

Sample dilution solution, pH (5.5 $\pm$ 0.2). About 4.1 g of anhydrous sodium acetate and 0.5 g of bovine serum albumin are placed in a 1-liter beaker and dissolved in 700 mL of purified water. The pH of the solution is adjusted to (5.5 $\pm$ 0.2) with acetic acid. The resulting solution is transferred to a 1-liter measuring flask, the volume of the solution is adjusted to the mark with purified water, mixed, and filtered through a membrane filter with a pore diameter of 0.45 $\mu$m.

Shelf life: 3 months at a temperature of 2 to 8°C.

Substrate dilution solution, pH (5.00 $\pm$ 0.05). 4.1 g of anhydrous sodium acetate, 1.9 g of lead diacetate trihydrate are placed in a 500 mL beaker,, 450 mL of purified water is added, and stirred until dissolved. The pH of the solution is brought to (5.00 ± 0.05) with acetic acid (about 1.1 mL). The resulting solution is transferred to a 500 mL volumetric flask, the volume of the solution is brought to the mark with purified water, stirred and filtered through a membrane filter with a pore diameter of 0.45 μm.

Shelf life: 3 months at a temperature of 2 to 8°C.

Substrate solution (1.25 mmol/l). 0.83 mL of substrate dilution solution is added to the vial containing the substrate (4-methylumbelliferyl α-L-idopyranosiduronic acid 2-sulfate sodium salt) and mixed gently. The resulting solution is divided into 0.2 mL aliquots and frozen.

Shelf life is 3 months at a temperature not exceeding minus 70°C.

Solution of recombinant human α-L-iduronidase. 400 μl of purified water is added into a vial containing recombinant human α-L-iduronidase (10 μg) and mixed. The resulting solution is divided into 0.1 mL aliquots and frozen.

Shelf life is 3 months at a temperature not exceeding minus 70°C.

0.1 M citric acid solution. About 19.2 g of anhydrous citric acid is placed in a 1 L measuring flask, dissolved in 900 mL of purified water, the volume of the solution is brought up to the mark with purified water, and filtered through a 0.22 μm membrane filter.

Shelf life: 3 months at a temperature of 15 to 25°C.

0.2 M solution of sodium phosphate dibasic. About 35.6 g of sodium phosphate dibasic dihydrate is placed in a 1 L measuring flask, dissolved in 900 mL of purified water, the volume of the solution is brought up to the mark with purified water, filtered through a 0.22 μm membrane filter.

Shelf life: 3 months at a temperature of 15 to 25°C.

Phosphate-citrate buffer solution, pH (4.5 ± 0.1). In a 100 mL volumetric flask, 55.0 mL of 0.1 M citric acid solution and 45.0 mL of 0.2 M sodium phosphate dibasic solution are mixed and filtered through a membrane filter with a pore diameter of 0.22 μm.

Shelf life: 3 months at a temperature of 15 to 25°C.

0.5 M sodium bicarbonate solution. About 42.0 g of sodium bicarbonate is placed in a 1 L measuring flask, dissolved in 900 mL of purified water, and the volume of the solution is brought up to the mark with purified water , and mixed, and filtered through a membrane filter with a pore diameter of 0.22 μm.

Shelf life: 6 months at a temperature of 15 to 25°C.

0.5 M sodium carbonate solution. About 53.0 g of sodium carbonate is placed in a 1 L measuring flask, dissolved in 900 mL of purified water, the volume of the solution is brought up to the mark with purified water, and mixed, and filtered through a membrane filter with a pore diameter of 0.22 μm.

Shelf life: 6 months at a temperature of 15 to 25°C.

Stop solution, pH (10.8 ± 0.5). 900 mL of 0.5 M sodium carbonate solution and 100 mL of 0.5 M sodium bicarbonate solution are added to a 1 L measuring flask, mixed and filtered through a membrane filter with a pore diameter of 0.22 μm.

Shelf life: 6 months at a temperature of 15 to 25°C.

Stock solution of 4-methylumbelliferone sodium salt (100 μmol/mL). About 1.0 g (accurately weighed) of 4-methylumbelliferone sodium salt is placed in a 50 mL measuring flask, 30 mL of purified water is added, mixed, and brought up to the mark with purified water. The solution is divided into aliquots of 2.0 μL.

Shelf life is 6 months at a temperature not exceeding minus 18°C.

Working standard solution of 4-methylumbelliferone sodium salt (50 nmol/mL).

An aliquot of the stock solution of 4-methylumbelliferone sodium salt is kept in a water bath for 5 minutes at 37 °C. Serial dilutions are prepared according to the following scheme:

| Solution No. | 4-MU* stock solution | Solution No. 2 | Stop solution | Concentration, nmol/mL |
|---|---|---|---|---|
| 1 | 100 | - | 9900 | 1000 |
| 2 | - | 500 | 9500 | 50 |
| *4-MU is 4-methylumbelliferone sodium salt. | | | | |

Dilutions of the working standard solution of 4-methylumbelliferone sodium salt. Dilutions of the working standard solution of 4-methylumbelliferone sodium salt are prepared according to the following scheme:

| Dilution No. | Working standard solution volume (50 nmol/mL), mL | Stop solution volume, mL | Concentration, nmol/mL |
|---|---|---|---|
| 1 | 0 | 5.00 | 0.0 |

(continued)

| Dilution No. | Working standard solution volume (50 nmol/mL), mL | Stop solution volume, mL | Concentration, nmol/mL |
|---|---|---|---|
| 2 | 0.05 | 4.95 | 0.5 |
| 3 | 0.10 | 4.90 | 1.0 |
| 4 | 0.15 | 4.85 | 1.5 |
| 5 | 0.20 | 4.80 | 2.0 |
| 6 | 0.25 | 4.75 | 2.5 |
| 7 | 0.30 | 4.70 | 3.0 |
| 8 | 0.35 | 4.65 | 3.5 |
| 9 | 0.40 | 4.60 | 4.0 |

Dilutions of the test sample. The test sample is diluted with sample diluent to a concentration of 1 mg/mL, based on the actual HIR-FAB-IDS content specified in the certificate. Serial dilutions are then prepared according to the following scheme:

| Tube No. | HIR-FAB-IDS concentration, ng/mL | Solution volume, μl | | | | | | | | |
|---|---|---|---|---|---|---|---|---|---|---|
| | | HIR-FAB-IDS solution (1 mg/mL) | Tube No. | | | | | | | Sample dilution solution |
| | | | 1 | 2 | 3 | 4 | 5 | 6 | 7 | |
| 1 | 100000 | 100 | - | - | - | - | - | - | - | 900 |
| 2 | 10000 | | 100 | - | - | - | - | - | - | 900 |
| 3 | 1000 | | | 100 | - | - | - | - | - | 900 |
| 4 | 100 | | | | 100 | - | - | - | - | 900 |
| 5 | 10 | | | | | 100 | - | - | - | 900 |
| 6 | 5 | | | | | | 100 | - | - | 100 |
| 7 | 2,5 | | | | | | | 100 | - | 100 |

All dilutions are kept on ice until testing begins.
In further studies, solutions from test tubes No. 5-7 are used.

*Conducting an analysis*

First enzymatic reaction

10 μl of each dilution of the test sample (in 2 replicates) is added to the wells of the plate; the sample dilution solution is used as a comparison solution. 20 μl of the substrate solution (4-methylumbelliferyl α-L-idopyranosiduronic acid 2-sulfate sodium salt) is added. The plate is covered with a film and incubated in a thermostatted shaker for 4 hours at a temperature of $(37.0 \pm 0.2)°C$ and a stirring speed of 250 rpm. The plate is protected from light throughout the incubation period.

Second enzymatic reaction

After incubation, 20 μl of phosphate-citrate buffer solution is added to the wells of the plate to stop the 1st enzymatic reaction. Then 10 μl of recombinant α-L-iduronidase solution is added to each well and mixed gently. The plate is covered with a film and incubated in a thermostatted shaker for 22-26 hours at 37°C and a mixing speed of 250 rpm. The plate is protected from light during the entire incubation period.
To stop the reaction, 200 μl of stop solution is added into each well with the incubated mixture.
260 μl of dilutions of the working standard solution of 4-methylumbelliferone sodium salt are added to empty wells of the plate.

In the wells of the plate, the fluorescence signal is measured at an excitation wavelength of 365 nm and a detection wavelength of 460 nm.

Evaluation of results

Based on the results of measuring the fluorescence signal in wells with dilutions of the working standard solution of 4-methylumbelliferone sodium salt, a graph of the linear relationship between the fluorescence signal and the concentration of 4-methylumbelliferone sodium salt (nmol/mL) is plotted. Using the equation of the linear function, the concentration of 4-methylumbelliferone produced during the reaction in wells with dilutions of the test samples and the comparison solution (nmol/mL) is calculated.

Specific activity A, in U/$\mu$g, for each dilution of the test samples is calculated using the formula:

$$A = 0{,}26 \times \frac{(m - m_0) \times 1000}{240 \times C \times 0{,}01}$$

where: m is the concentration of 4-methylumbeliferone produced during the reaction in wells with a given dilution of the test sample (average value over 2 replicates), nmol/mL; $m_0$ is the concentration of 4-methylumbeliferone produced during the reaction in wells with the comparison solution (average value over 2 replicates), nmol/mL;

0.26 is the final volume of dilutions of the working standard solution and the test sample added to the wells of the plate to measure the fluorescence signal, mL;

240 is the time of the 1st enzymatic reaction, min;

C is the content of HIR-Fab-IDS in each of the prepared dilutions of the test sample, ng/mL;

0.01 is the volume of dilutions of the test sample added into the 1st enzymatic reaction, mL;

1000 is the conversion factor from ng to $\mu$g.

The final value of specific activity for the test sample is calculated as the average value of specific activity calculated for each dilution.

The results obtained for determining the enzymatic activity of the HIR-FAB-IDS and HIR-MAB-IDS variants in comparison with the drug Elaprase® are presented in Table 2.

Table 2. Evaluation of iduronate-2-sulfatase activity of HIR-FAB-IDS variants and HIR-MAB-IDS against unmodified iduronate-2-sulfatase enzyme (Elaprase®)

| Sample name | Activity, units (nmol/min)/$\mu$g |
| --- | --- |
| HIR-FAB-IDS (SEQ ID NO:2 and SEQ ID NO:4) | 27.0 |
| HIR-FAB-IDS (SEQ ID NO:2 and SEQ ID NO:5) | 20.0 |
| HIR-FAB-IDS (SEQ ID NO:8 and SEQ ID NO:12) | 25.2 |
| HIR-FAB-IDS (SEQ ID NO:9 and SEQ ID NO:13) | 30.2 |
| HIR-FAB-IDS (SEQ ID NO:10 and SEQ ID NO:14) | 27.5 |
| HIR-FAB-IDS (SEQ ID NO:11 and SEQ ID NO:15) | 23.4 |
| HIR-MAB-IDS | 11.0 |
| HIR-MAB-IDS (AGT-182) (value taken from patent US8834874 B2 disclosing the drug AGT-182 (see Fig. 12 example 4, p. 35) | 0.85 (51.7+-7nmol/hour/$\mu$g) US8834874 B2 |
| Elaprase® (Shire Human Genetic Therapies Inc., USA) | 14.6 |

As can be seen from the results presented in Table 2, the HIR-FAB-IDS preparation exhibits specific enzymatic (iduronate-2-sulfatase) activity towards the substrate 4-MU-$\alpha$IdoA-2S (4-methylumbelliferyl $\alpha$-L-idopyranosiduronate-2-sulfate) 27.0 U/$\mu$g. At the same time, the free recombinant enzyme iduronate-2-sulfatase exhibits an activity of 14.6 U/$\mu$g. The full-length antibody to the insulin receptor, coupled with the amino acid sequence of iduronate-2-sulfatase (HIR-MAB-IDS), exhibits lower specific activity (11.0 U/$\mu$g), in comparison with HIR-FAB-IDS.

The conducted studies showed that iduronate-2-sulfatase in HIR-FAB-IDS retains the main functional (enzymatic) properties at the level of the free recombinant enzyme. At the same time, the specific activity of HIR-FAB-IDS is higher than that of the drug Elaprase® and HIR-MAB-IDS (AGT-182).

Thus, an increase in the enzymatic activity of a compound containing a transport element, which is a Fab fragment of immunoglobulin IgG, specific to an epitope in the insulin receptor, connected directly or via a linker to a therapeutic enzyme, was detected, in comparison with a therapeutic enzyme without a transport element, and with a therapeutic enzyme with a transport element represented by MAB.

### Example 3. Study of the efficacy of the HIR-Fab-IDS preparation in peripheral tissues of 24744 line mice: B6N.Cg-IdstmlMuen/J IDS KO

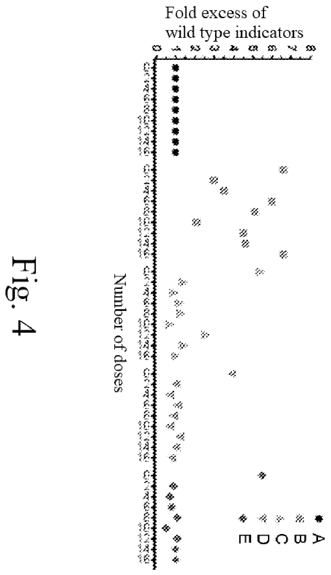

In the HIR-Fab-IDS efficacy study, sixty (60) hemizygous male iduronate-2-sulfatase knockout mice B6N.Cg-IdstmlMuen/J (JAX line #024744) and twelve (12) wild-type (C57BE/6NJ) animals were used as controls. Animals were randomized based on age at first dosing (11 weeks) and were treated weekly with HIR-Fab-IDS at doses of 0.3, 1.0, and 3.0 mg/kg and saline as a control for 16 weeks. Blood and urine samples were collected during dosing. Knockout mice in the control group demonstrated 4.7-fold higher urinary GAG levels than normal animals. As shown in Fig. 4, in the HIR-Fab-IDS dosing groups, urinary GAG levels were reduced to normal animal levels after the first dosing and were maintained throughout the dosing period.

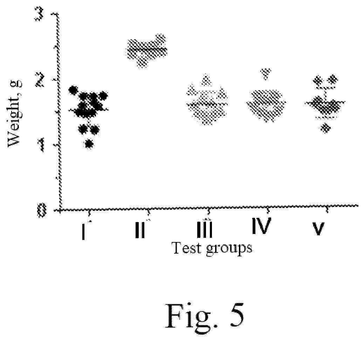

One hour after the last administration, the animals were euthanized, and terminal blood and tissue samples were collected. Histological analysis of the tissues was performed, as well as measurement of the iduronate-2-sulfatase activity level and GAG accumulation in the tissues of the experimental animals. The liver weight in the knockout animals in the control group was 1.5 times higher than in the wild-type animals. Following administration of the drug, the liver weight in the knockout mice of the dosing groups returned to normal (Fig. 5).

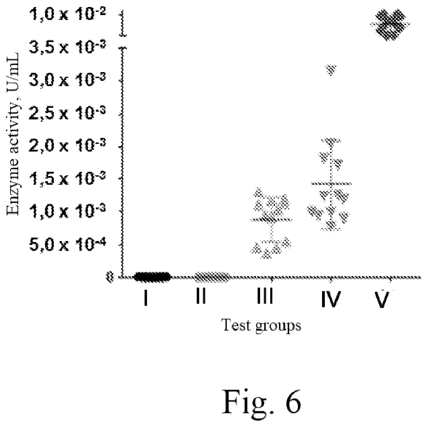

The level of iduronate-2-sulfatase activity in the plasma of knockout animals differed significantly (-70%) from wild-type animals. In 1 h after administration, all HIR-Fab-IDS dosing groups demonstrated significantly higher values of this parameter than knockout animals of the control group (263-2408 times), as well as wild-type animals (80-730 times). Mice receiving 0.3 mg/kg of HIR-Fab-IDS compound had, on average, 263-fold excess of iduronate-2-sulfatase activity compared to knockout animals receiving saline. The results of the analysis of iduronate-2-sulfatase activity in the plasma of experimental animals are presented in Fig. 6.

The level of iduronate-2-sulfatase activity in the tissues of the main organs (spleen, liver, kidneys and heart) of knockout animals that did not receive drug therapy was significantly (on average by 76%) reduced compared to normal animals. Knockout animals of all dosage groups demonstrated significantly higher (on average by 17-56 times) values of iduronate-2-sulfatase enzymatic activity one hour after drug administration compared to the group of animals that received saline, and exceeded those in wild-type animals by 2.6-10 times. The HIR-Fab-IDS compound did not have a significant effect on the level of enzyme activity in the brain tissues of knockout animals when administered at low and medium doses. In the high dose group, iduronate-2-sulfatase activity was increased in the brain tissue of knockout animals by an average of 50% compared to the placebo group, but was 82% lower compared to wild-type animals.

GAG levels in the tissues of the main organs of knockout animals that did not receive the drug were significantly (on average 12 times) higher than that in normal animals. Knockout animals of all dosage groups demonstrated significantly lower (on average 70%) GAG levels compared to the group of animals that received saline (on average -70% in all tissues and dose groups) and slightly (1.0-1.5 times in all tissues and dose groups) exceeded the values of this indicator in normal animals. The levels of GAG in brain tissue did not differ between knockout and normal animals, as well as between knockout animals that received the drug and the control group (receiving saline).

Thus, 16-week administration of the drug to iduronate-2-sulfatase gene knockout mice at doses of 0.3-3 mg/kg corrected the increase in GAG levels in urine and tissues of major organs, and normalized the increase in liver weight to the level of wild-type animals. One hour after dosing, animals of all three dosing groups demonstrated an increase in the level of iduronate-2-sulfatase activity in plasma and tissues of major organs (except for the brain) compared to the values in the knockout animals of the control group (263 to 2408-fold excess). In the brain tissues of knockout animals of the dosing groups, such a correction did not reach the values obtained for wild-type animals. The efficacy of HIR-Fab-IDS was demonstrated at all doses studied in the mouse model of MPS II (Hunter syndrome).

### Example 4. Analysis of tissue distribution of radioactively labeled Fab fragments of an antibody to the insulin receptor linked to the amino acid sequence of iduronate-2-sulfatase [125I]-HIR-Fab-IDS (SEQ ID NO:2 and SEQ ID NO:4), [125I]-HIR-Mab-IDS, [125I]-IDS (control) after intravenous administration to cynomolgus macaques

The experimental animals were administered a single dose of the respective test molecule at a dose of 2 mL/kg body weight by intravenous bolus injection over 1-2 minutes (or 30 seconds) via the left femoral vein. The target radioactive dose level was 1 MBq/kg animal body weight for all studied molecules.

The animal that received the test substance was sedated with an intramuscular injection of ketamine hydrochloride before an intravenous overdose of Doletil (pentaborbitone sodium). The animals were humanely euthanized 2 hours after administration of the test substance. During sedation, 2 mL of blood was collected from the cephalic vein of each animal and centrifuged to obtain plasma. The concentration of radioactivity in the animals' blood plasma was measured. After death was confirmed, each animal was snap-frozen in a mixture of solid carbon dioxide in hexane. After complete freezing, the bodies were placed in a mold containing 2% (w/v) aqueous carboxymethylcellulose paste. Several longitudinal sagittal sections (nominally 30 $\mu$m) were made at least at 5 body levels and, if necessary, 3 head levels for each individual.

Sections mounted on Filmolux 610 tape (Neschan) were lyophilized in a GVD03 tabletop freeze dryer (Girovac Ltd) and applied to FUJI imaging plates (type B AS-MS, Raytek Scientific Ltd). [125I]-labeled blood standards of appropriate activity, also prepared as sections of nominal thickness 30 $\mu$m, were applied to the imaging plates.

After development in a copper-lined lead box for 7 days, the radiographic plates were processed using a FUJI FLA-5000 radiographic system (Raytek Scientific Ltd). The electronic images were analyzed using a PC-based image analysis system (Seescan 2 software, LabLogic Ltd). Standards [125I] included with each autoradiogram were used to plot calibration curves over a range of radioactivity concentrations.

The concentration of radioactivity in plasma was determined by gammametry.

Tissue concentration data were recorded as lg equivalents of [125I] HIR-Fab-IDS [125I] HIR-Mab-IDS and [125I]-IDS (control).

The results were expressed to three significant figures with no more than two decimal places. The data tables were computer generated and individual data were appropriately rounded.

[125I] HIR-Fab-IDS, [125I] HIR-Mab-IDS and [125I]-IDS (control) were administered to male cynomolgus monkeys as single intravenous doses at nominal dose levels of 0.0020 mg/kg, 0.0015 mg/kg and 0.0010 mg/kg body weight. The doses varied because each solution contained different levels of radioactivity, ensuring a uniform level of 0.0015 mg/kg body weight. Animals were euthanized 2 h after each administration and frozen for whole body autoradiography studies.

Following intravenous administration, the drugs were absorbed and widely distributed throughout the body tissues, and at the time of sacrifice, all tissues studied had been exposed to them. The lower limit of quantification was 0.2, 0.03 and 0.097 ng drug equivalent/g for all animal tissue concentration measurements, respectively.

The plasma and blood radioactivity concentrations of the animal treated with [125I] HIR-Fab-IDS were 2.55 and 3.11 ng eq/g, respectively (blood:plasma ratio 1.22). Quantifiable levels of radioactivity were present in several brain regions including the medulla oblongata (1.09 ng eq/g), cerebral cortex (1.03 ng eq/g), cerebellum (0.908 ng eq/g), hypothalamus (0.869 ng eq/g), cerebellar dendritic substance (0.799 ng eq/g), pons (0.793 ng eq/g), and medulla (0.562 ng eq/g); the TP ratios ranged from 0.22 to 0.43.

The concentrations of radioactivity in plasma and blood of the animal treated with [125I] HIR-Mab-IDS were 2.13 and 1.86 ng eq/g, respectively (blood:plasma ratio 0.87). Quantitative levels of radioactivity were present in a number of brain regions of the animal. These regions included the medulla oblongata (0.803 ng eq/g), cerebellar dendritic substance (0.606 ng eq/g), cerebellum (0.494 ng eq/g), cerebral cortex (0.453 ng eq/g), pons (0.438 ng eq/g), medulla (0.288 ng eq/g), and hypothalamus (0.279 ng eq/g), with TP concentration ratios ranging from 0.13 to 0.38.

Plasma and blood concentrations in the animal receiving [125I]IDS were 0.910 and 0.753 ng Eq/g, respectively (blood:plasma ratio 0.83). Quantitative levels of radioactivity were present in the whole brain (0.113 ng Eq/g), but concentrations were below the limit of quantification (0.097 ng Eq/g) in all regions examined: medulla oblongata, cerebral cortex, cerebellum, hypothalamus, cerebellar dendritic substance, pons, and medulla. Concentrations in CNS tissues were significantly lower than in plasma and blood.

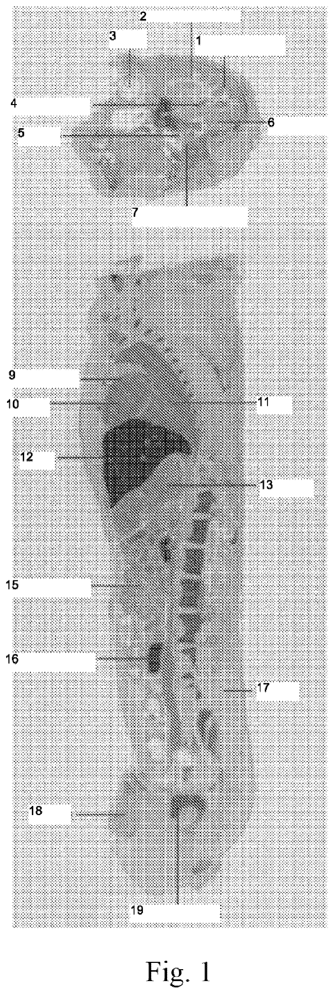

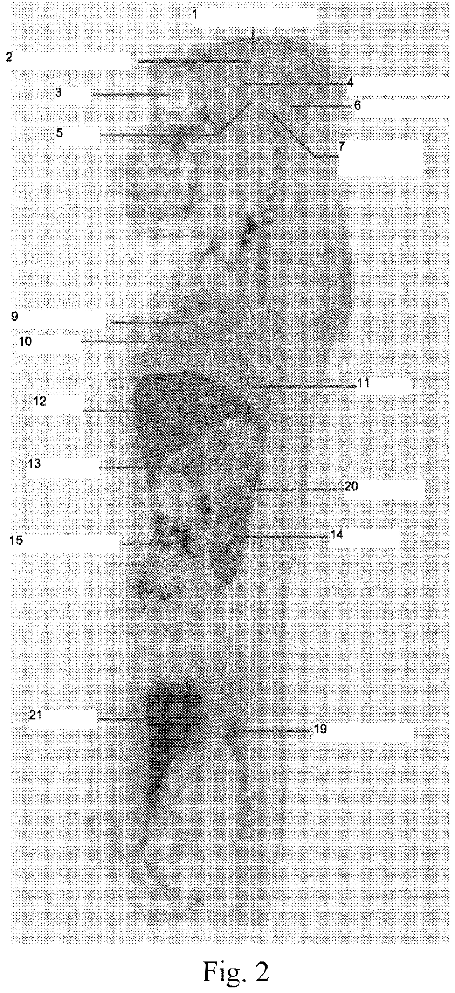

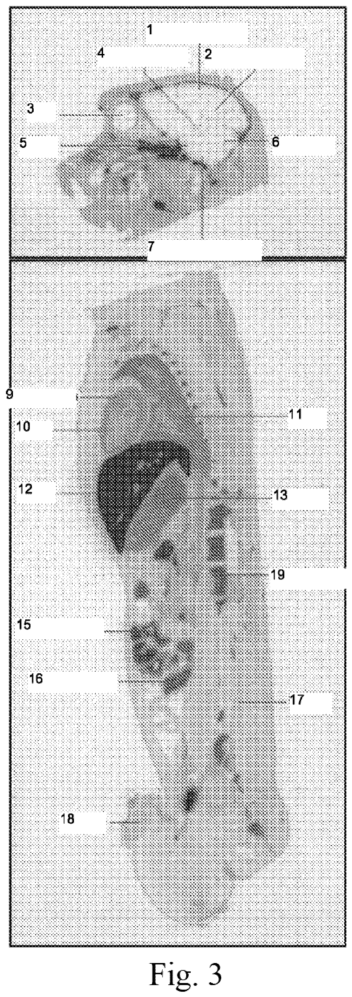

These results indicate that the HIR-Fab-IDS-bound and HIR-Mab-IDS-bound substances penetrated the blood-brain barrier compared to the IDS-bound substance, CNS tissues were exposed to the test substance or its labeled metabolites, and blood-borne radioactivity had little effect on the determination of CNS tissue concentrations (Figs. 1 and 2). However, HIR-Fab-IDS exhibited a broader distribution in body tissue compared to the HIR-Mab-IDS molecule (Table 5, Fig. 3).

It should be noted that, in example 10 of invention US8834874 B2 (AGT-182 or HIR-MAB-IDS), the efficiency of penetration into the human brain is assessed at a level of 1% of the administered dose per 1000 grams of brain tissue based on experimental data on the penetration of AGT-182 (HIR-MAB-IDS) into the brain in rhesus macaques. It is claimed that at this level of penetration, 20% of the iduronate-2-sulfatase (IDS) activity in the human brain is expected to be achieved, which will eliminate the accumulation of glycosaminoglycans in the brain of a patient.

Taking into account the obtained data on penetration into the brain of cynomolgus macaques (Table 5), it can be concluded that the present compound penetrates into the brain of cynomolgus macaques almost 2 times better than HIR-MAB-IDS, i.e. 0.84 ng equiv/g HIR-FAB -IDS versus 0.43 ng equiv/g for HIR-MAB-IDS. This accordingly means that HIR-FAB-IDS penetrates into the human brain with 2% efficiency. Taking into account the calculations specified in Example 10 of invention US8834874B2, it is expected to achieve 40% of the iduronate-2-sulfatase activity in the human brain, without taking into account the increased activity of HIR-FAB-IDS in comparison with HIR-MAB-IDS (AGT- 182), see Table 2.

If we take into account the fact of the increase in the specific activity of HIR-FAB-IDS over HIR-MAB-IDS, shown in Example 2, Table 2 - 27 U/mcg for the real compound and 11 U/mcg for the contrasted one, as well as greater penetration into the brain: 27/11 = 2.4 times higher specific iduronate-2-sulfatase activity, 0.84/0.43 = 1.95 times greater penetration into the brain, then we get 20% restoration of IDS activity in the brain of a patient with HIR-MAB-IDS therapy and 20% $\times$ 2.4 $\times$ 1.95 = 93.6% restoration of iduronate-2-sulfatase activity in the brain of a patient with MPS type II from the level of IDS activity in the brain of a healthy person. Thus, the present invention will allow almost complete restoration of the enzymatic function of IDS in the brain of a patient, while the contrasting HIR-MAB-IDS will only restore it by 20% (93% vs. 20%).

Thus, it was found that the administration of a compound containing a transport element, which is a Fab fragment of immunoglobulin IgG, specific to an epitope in the insulin receptor, connected directly or via a linker to a therapeutic enzyme, provides a higher degree of substitution of the activity of the therapeutic enzyme in the human brain in comparison with compounds containing Mab.

Table 5. Radioactivity concentrations in cynomolgus macaque tissues after a single intravenous administration of [125I]- HIR- Fab-IDS

| Tissue | Tissue radioactivity concentrations | | |
|---|---|---|---|
| | [125I] HIR-Fab-IDS (SEQ ID NO:2 and SEQ ID NO:4) | [125I] HIR-Mab-IDS | [125I]-IDS |
| Plasma | 2.55 | 2.13 | 0.91 |
| Blood | 3.11 | 1.86 | 0.75 |
| Adrenal cortex | 18.3 | 5.29 | 4.47 |
| Adrenal medulla | 11.5 | 2.17 | 3.26 |
| Aortic wall | 3.29 | 1.96 | BLQ |
| Bone marrow | 6.37 | 3.26 | 4.39 |
| Periosteum | 3.32 | BLQ | 0.95 |
| Brown fat | 2.51 | 0.97 | 0.54 |
| Bulbourethral gland | 0.98 | BLQ | 0.60 |
| Mucosa of cecum | 1.72 | 0.19 | 0.25 |
| Epididymis | 3.09 | 0.31 | 0.53 |
| Renal cortex | 10.1 | 4.25 | 4.33 |
| Renal medulla | 6.37 | 2.39 | 1.70 |
| Mucosa of colon | 1.42 | 0.41 | 0.24 |
| Liver | 21.4 | 7.63 | 18.0 |
| Lung | 4.15 | 1.10 | 2.35 |
| Muscles | 0.51 | 0.09 | 0.28 |
| Myocardium | 3.78 | 0.91 | 1.25 |
| Mucosa of nose | 1.18 | 0.61 | 0.72 |
| Wall esophagus | 1.53 | 0.51 | 0.48 |
| Pancreas | 2.68 | 0.70 | 0.69 |
| Mucosa of rectum | 1.15 | 0.39 | 0.27 |
| Salivary glands | 1.01 | 0.23 | 0.82 |
| Seminal glands | 2.08 | BLQ | BLQ |
| Skin | 1.20 | 0.20 | 0.39 |
| Mucosa of small intestine | 3.14 | 0.62 | 1.62 |
| Spinal cord | 0.57 | 0.41 | 0.17 |
| Spleen | 17.9 | 10.4 | 11.6 |
| Testicles | 1.34 | 0.18 | 0.41 |

(continued)

| Tissue | Tissue radioactivity concentrations | | |
|---|---|---|---|
| | [125I] HIR-Fab-IDS (SEQ ID NO:2 and SEQ ID NO:4) | [125I] HIR-Mab-IDS | [125I]-IDS |
| Tongue | 0.92 | 0.30 | 0.64 |
| Trachea | 0.65 | 0.32 | 0.45 |
| Wall of urinary bladder | 7.30 | 3.49 | 4.18 |
| Brain (whole) | 0.84 | 0.43 | 0.11 |
| Cerebral cortex | 1.03 | 0.45 | BLQ |
| Medule | 0.56 | 0.29 | BLQ |
| Cerebellum | 0.91 | 0.49 | BLQ |
| Hypothalamus | 0.87 | 0.28 | BLQ |
| Medulla oblongata | 1.09 | 0.80 | BLQ |
| Pons | 0.79 | 0.44 | BLQ |
| Cerebellar dendritic substance | 0.80 | 0.61 | BLQ |
| Pineal gland | 1.03 | 0.39 | BLQ |
| Pituitary gland | 0.80 | 0.60 | BLQ |
| NA - Not applicable; BLQ - Concentration below the lower limit of quantification | | | |

The present description represents compounds containing therapeutic enzymes that exhibit their specific enzymatic activity in said compounds, and transport elements capable of interacting with the insulin receptor, while having the ability to transport therapeutic enzymes to tissue lysosomes, including through the BBB to lysosomes of nervous tissue. Thus, the compounds exhibit high activity and an improved ability to be transported to lysosomes of tissue cells of various organs, including lysosomes of nervous tissue cells, which suggests the use of the present invention for enzyme replacement therapy for treatment or prophylaxis in a subject with a lysosomal storage disease.

**Example 5. Ingredients of a pharmaceutical composition**

The pharmaceutical compositions of the present invention, which are used for therapeutic or prophylactic purposes, can be prepared by mixing, if necessary, with suitable pharmaceutically acceptable carriers, excipients, etc., and preparing them in the form of freeze-dried preparations or preparations in the form of a solution. Examples of suitable pharmaceutically acceptable carriers and excipients include sterilized water, physiological saline solution, stabilizers, excipients, antioxidants (such as ascorbic acid), buffers (such as phosphate, citrate, histidine buffer or buffers based on other organic acids), antiseptics, surfactants (such as PEG and Tween), chelating agents (such as EDTA) and binders. They may also contain other low molecular weight polypeptides, proteins such as serum albumin, gelatin and immunoglobulins, amino acids such as glycine, glutamine, asparagine, glutamic acid, aspartic acid, methionine, arginine and lysine, sugars and carbohydrates such as polysaccharides and monosaccharides, and sugar alcohols such as mannitol and sorbitol. In preparing an aqueous injection solution, for example, physiological saline and isotonic solutions containing glucose and other adjuvants such as D-sorbitol, D-mannose, D-mannitol and sodium chloride and appropriate stabilizers such as alcohol (e.g. ethanol), polyhydric alcohols (such as propylene glycol and PEG) and nonionic surfactants (such as polysorbate 80, polysorbate 20, poloxamer 188 and HCO-50) can be used in combination with each other. In the case where hyaluronidase is mixed with the preparation, large volumes of liquid can be administered subcutaneously (Expert Opin. Drug Deliv., 4(4), July 2007; pp. 427-440). In addition, the pharmaceutical compositions of the present invention can be preloaded into a syringe. For this purpose, the preparation can be obtained in the form of a solution according to the method described in WO2011/090088.

If desired, the antigen-binding molecules of the present invention can be incorporated into microcapsules (e.g., composed of hydroxymethylcellulose, gelatin, and poly(methyl methacrylate)) or incorporated into colloidal drug delivery systems (e.g., liposomes, albumin microspheres, microemulsions, nanoparticles, and nanocapsules) (see, e.g., Remington's Pharmaceutical Science, 16th edition, Oslo, 1980). Methods for preparing pharmaceuticals in the form of controlled release pharmaceuticals are also well known and such methods can be applied to the antigen binding molecules of the present invention (Langer et al., J. Biomed. Mater. Res., 15, 1981, pp. 267-277; Langer, Chemtech., 12, 1982, pp.

98-105; US 3,773,919; European Patent Application Publication EP 58481; Sidman et al., Biopolymers, 22, 1983, pp. 547-556; EP 133988).

Pharmaceutical composition of concentrate for preparation of solution for infusions:

Compound HIR-FAB-IDS 5.3 mg
Sodium chloride 8.0 mg
Sodium dihydrogen phosphate dihydrate 3.12 mg
Sodium hydroxide to adjust pH to pH 6.0
Polysorbate 20-0.2 mg
Water for infusions q.s. to 1.0 mL

**Example 6. Calculation and justification of the starting dose for the first use in humans**

The calculation and justification of the starting dose for the first use in humans were based on the NOAEL (No Observed Adverse Effect Level) and MABEL (Minimal Anticipated Biological Effect Level; dose that has a minimum expected biological effect) approaches, since the phase I study planned a single administration of the drug to healthy volunteers within the expected therapeutic range without titration to the maximum tolerated dose. Information on the efficacy and safety of drugs with similar mechanisms of action in humans was also taken into account.

The main results of the efficacy and safety assessment in preclinical studies, which reflect the risk-benefit criterion, are presented in Table 6.

Table 6. Extrapolation to humans of doses obtained as a result of the study of the efficacy and general toxic effect of the HIR-FAB-IDS compound in adult animals

| Test | HIR-FAB-IDS compound dose in different animal species, mg/kg | | | Extrapolation to a 70 kg human, mg/kg* |
|---|---|---|---|---|
| | Mouse | Rat | Cynomolgus macaque | Human |
| Nonatoxic dose with single administration (IV) | | 30 | 30 | 30 |
| Nonatoxic dose with repeated administration (IV, once a week, 8 weeks) (NOAEL) | | 30 | 30 | 30 |
| Minimum pharmacologically active dose in a mouse model of MPS II (IV, once a week, 16 weeks) | 0.3 | | | 0.3 |
| * dose extrapolation to a 70 kg human was performed without using interspecies dose transfer coefficients based on body surface area. | | | | |

In safety studies of the HIR-FAB-IDS compound, it was found that in monkeys, when administered weekly for 8 weeks at doses of 3, 10, and 30 mg/kg, none of the doses caused adverse effects. In rats, at weekly administration for 8 weeks at doses of 3, 10 and 30 mg/kg, none of the studied doses caused adverse effects. Thus, the highest non-toxic dose confirmed with 8-9-fold weekly administration in rodents and non-rodents is a dose of 30 mg/kg. The therapeutic index in preclinical studies, defined as the ratio of the highest safe dose (30 mg/kg) to the effective dose with multiple course administration (0.3 mg/kg) for the HIR-FAB-IDS compound, is 30 mg/kg / 0.3 mg/kg = 100.

To extrapolate the doses listed in the table to humans, no interspecies dose transfer coefficients based on body surface area were used because the molecular weight of HIR-FAB-IDS exceeds 100 kDa. Therefore, the human equivalent maximum safe dose (HED NOAEL) is 30 mg/kg. From a risk minimization perspective, a starting dose of 0.3 mg/kg should be adopted in clinical trials, as it has been shown in vivo to cause minimal pharmacological effect and is safe.

To improve the safety of starting dose prediction in humans, a calculation based on the MABEL approach was also performed. As a result of our in vitro pharmacodynamic studies, the lowest concentration of the HIR-FAB-IDS compound that has a biological effect is detected in the test for determining the activity of iduronate-2-sulfatase in fibroblasts of a patient with MPS II after treatment with the HIR-FAB-IDS compound. In this test, the threshold increase in the activity of iduronate-2-sulfatase (~20%) is detected at a concentration of $5 \times 10^{-9}$ M of the HIR-FAB-IDS compound. Given the molecular weight of the HIR-FAB-IDS compound (105 kDa), this corresponds to a concentration of the HIR-FAB-IDS compound equal to $5.25 \times 10^{-4}$ g/L. This concentration can be considered as a starting concentration for humans, to achieve which in the blood of an average human weighing 70 kg and with a fluid volume in the tissues of 56 liters (80%) it is necessary to administer 56 L $\times$ 5.25$\times10^{-4}$ g/L = 294$\times10^{-4}$ g of the HIR-FAB-IDS compound (which will correspond to 4.2$\times10^{-4}$ g/kg or 0.42 mg/kg). Therefore, based on in vitro data, the MABEL approach made it possible to determine the starting effective dose of the HIR-FAB-IDS compound in humans of at least 0.42 mg/kg.

At the same time, the results of pharmacodynamic (PD) studies in vivo obtained on B6N.Cg-IdstmlMuen/J mice (JAX line No. 024744) knocked out for the iduronate-2-sulfatase gene indicate that the lowest dose of the HIR-FAB-IDS compound, which causes a pharmacodynamic effect (reduces the content of glycosaminoglycans in tissues) is 0.3 mg/kg.

Thus, based on the in vitro data, the MABEL approach determined the starting dose of HIR-FAB-IDS in humans to be at least 0.42 mg/kg, and based on the in vivo data, the MABEL approach determined the starting dose of HIR-FAB-IDS in humans to be at least 0.3 mg/kg. According to the existing regulations, the lower of the specified values should be taken as the starting dose in this case, i.e. the starting effective dose of HIR-FAB-IDS for use in humans should be at least 0.3 mg/kg.

Thus, the choice of dose for the first use of the drug in humans was based on the following provisions (Table 7):

Table 7. Important data and results from preclinical studies guiding dose selection for human studies

| No. | Own studies and published materials | Results |
| --- | --- | --- |
| 1 | Recommended therapeutic dose of Elaprase® drug | 0.5 mg/kg once a week. |
| 2 | Study of specific activity | A slightly higher molar activity (ratio 1.26) compared to idursulfase (Elaprase®) was shown. The molecular weight is 59.3 kDa for of idursulfase and 105 kDa for HIR-FAB-IDS. |
| 3 | Evaluation of the comparative activity of iduronate-2-sulfatase in fibroblasts of a patient with mucopolysaccharidosis type II after treatment with the compound HIR-FAB-IDS and Elaprase® drug | Similar biological activity of the compound HIR-FAB-IDS and Elaprase® drug. EC50 for the HIR-FAB-IDS compound in GM000690 fibroblast cells was 27.81 $\pm$ 3.66 nM, and for Elaprase® drug 26.33 nM. |
| 4 | Pharmacodynamic study of the drug in mice knocked out by the iduronate-2-sulfatase gene. | PD effect was demonstrated for doses of 0.3, 1.0 and 3.0 mg/kg, with statistical significance achieved at doses of 1.0 and 3.0 mg/kg. The minimum pharmacologically active dose is 0.3 mg/kg. |
| 5 | Ex vivo study of cytokine release in whole blood of healthy donors | Of the 12 cytokines analyzed, only an increase in IL-8 was recorded. At the same time, within the expected therapeutic dose range (0.3-3 mg/kg), the IL-8 level was statistically significantly lower than the positive control (LPC). |
| 6 | Repeated dose toxicity studies (rats aged 8 weeks, cynomolgus macaques aged 9 weeks) | NOAEL for both species was 30 mg/kg. Extrapolation to humans: maximum safe dose is 30 mg/kg. |
| 7 | Repeated dose toxicity studies (immature cynomolgus macaques aged 26 weeks) | NOAEL for immature animals was 10 mg/kg. Extrapolation to humans: 10 mg/kg. |
| 8 | Results of the study of the drug valanafusp alfa (iduronidase with an IgG domain to the insulin receptor) | During infusions at all dose levels, transient dose-dependent hypoglycemia was observed with a frequency of 6.4%, which did not limit the implementation of therapy. |

During infusions at all dose levels, transient dose-dependent hypoglycemia was observed with a frequency of 6.4%, which did not limit therapy.

Based on the above, the therapeutic dose in humans is expected to be in the range of 1-3 mg/kg, the minimum effective dose extrapolated to humans is 0.3 mg/kg, the penetration of the drug through the BBB is confirmed for doses of 0.0015 to 0.0020 mg/kg. The therapeutic index is 100.

The safe dose ranges of the drug calculated on the basis of preclinical safety data, reaching a maximum of 30 mg/kg of the drug for adults and 10 mg/kg for children with repeated administration, include the expected therapeutic dose range with at least a 10-fold safety factor. The pharmacologically active dose of 0.3 mg/kg was adopted as the starting dose for the first use in humans in the IDB-MPS-I phase I clinical study.

The drug can only be administered intravenously as a 3-hour infusion.

Administration of the drug should be carried out under the supervision of a physician or other medical personnel who has experience in the treatment of patients with MPS II or other inherited metabolic disorders. During the study, the condition of patients will be closely monitored, with their mandatory stay in the study center for at least 6 hours after the completion of the first infusion for each new dose level of the HIR-FAB-IDS compound and observation for the development of infusion reactions in patients. Detailed information on the dose of the drug, duration of infusion, preparation of the infusion solution and its method of use, as well as disposal of unused drug is provided in the Clinical Study Protocol.

Rationale for dose selection for phase II-III clinical study

Based on preclinical and toxicological data, the results of the phase I clinical study IDB-MPS-I, which demonstrated good tolerability and a favorable safety profile of the study drug HIR-FAB-IDS with a single administration in the dose range from 0.3 mg/kg to 3 mg/kg (0.3 mg/kg, 0.5 mg/kg, 1 mg/kg, 2 mg/kg and 3 mg/kg), and also taking into account the short half-life of the drug, the starting dose of the drug HIR-FAB-IDS for the first use in patients with MPS II can be determined as < 3 mg/kg.

The choice of a starting dose of 1 mg/kg is also due to the expected somatic effect of the HIR-FAB-IDS compound equivalent to the therapeutic effect of Elaprase®. The HIR-FAB-IDS compound does not differ from idursulfase in its interaction with M6PR, penetration into M6PR -presenting cells, and enzymatic activity in cell cultures of MPS II patients. The results of the studies show that plasma clearance of the HIR-FAB-IDS compound occurs primarily through interaction with the M6PR receptor, which will provide comparable exposure in M6PR cells in vivo.

The HIR-FAB-IDS compound reduces the GAG level in fibroblasts of MPS II patients to the level of healthy donors at a concentration of 120 nM. Taking into account the blood volume in children over 3 years of about 75 mL/kg and in adolescents about 70 mL/kg, an effective concentration of 120 nM can be achieved by administering the HIR-FAB-IDS compound at a dose of 0.9-1.0 mg/kg.

In the 26-week toxicity study, the NOAEL was defined as 10 mg/kg/week based on transient hypoglycemia, which is a predictable and expected class effect. Hypoglycemia has been described with other fusion proteins using the insulin receptor (HIR) to mediate drug trafficking across the BBB, including two HIR mAb products linked to iduronidase or idursulfase. A similar toxicity profile in preclinical studies (transient hypoglycemia at high doses up to 30 mg/kg) and isolated cases of dose-related hypoglycemia in patients with MPS II clinical trials confirm the safety of the selected starting dose of 1 mg/kg in children.

The starting dose of 1 mg/kg is also justified from an ethical point of view, as it allows avoiding the administration of subtherapeutic doses and reducing the already achieved effect of ERT.

**Example 7. Confirmation of the principle of action of the penetration of the HIR-Fab-IDS compound through the BBB**

Proof of principle was obtained in patient 1001, a participant in the IDS-MPS-II/III clinical trial.

Patient 1001 was an 18-year-old male. The diagnosis of MPS type II (Hunter disease) with a neurological component/neuropathic form was confirmed in 2010 by a decrease in the plasma iduronate-2-sulfatase content to 3.18 nM/4 h/mL (normal 297-705), and genotypically: in exon 09 of the IDS gene (OMIM 300823), a nucleotide substitution c.1327C>T was detected in the hemizygous state, leading to translation termination p.R443. The nucleotide substitution has not been described previously, but according to computer analysis (Alamut Visual, version 2.10), it may be pathogenic. Since 2011, the patient has been receiving enzyme replacement therapy with Elaprase® (INN idursulfase).

Before the start of therapy with the HIR-FAB-IDS compound, the patient's urinary glycosaminoglycan (GAG) excretion was close to normal due to enzyme replacement therapy. During treatment, urinary GAG excretion rates did not change significantly, which confirms comparable enzymatic activity of the drug in the periphery. The concentration of GAG in the cerebrospinal fluid (CSF) was initially high: 3294.5 ng/mL heparan sulfate (HS) and 3.5 ng/mL dermatan sulfate (DS). It has been shown experimentally that in MPS II, GS predominantly accumulates in the central nervous system (CNS), and the concentration of GS in the CSF correlates with its accumulation in brain tissue (Tanaka N., Kida S., Kinoshita M., Morimoto H., Shibasaki T., Tachibana K., Yamamoto R. Evaluation of cerebrospinal fluid heparan sulfate as a biomarker of neuropathology in a murine model of mucopolysaccharidosis type II using high-sensitivity LC/MS/MS. Mol. Genet. Metab. 2018 Sep.; 125(1-2):53-58. doi: 10.1016/j.ymgme.2018.07.013. Epub. 2018 Jul. 23. PMID: 30064964).

According to the protocol, the patient received the HIR-FAB-IDS compound as weekly intravenous infusions lasting 3 hours. After 3 administrations The dose was gradually increased from 1 to 3 mg/kg to assess safety and tolerability. During each first administration of the drug at a new dose, serum samples were collected for pharmacokinetic studies. Cerebrospinal fluid samples for drug content and GAG concentration were collected during screening (baseline) and after 3 weeks of drug administration at doses of 2 and 3 mg/kg. CSF collection for drug concentration assessment was performed 2.5 hours after the end of the infusion.

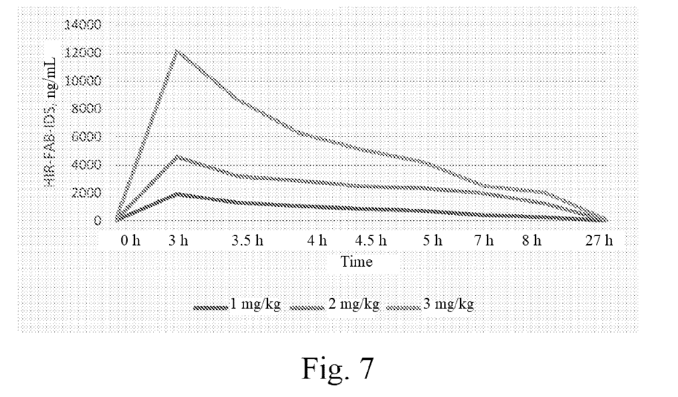

The pharmacokinetics of the drug is nonlinear. The values of the concentrations of the HIR-FAB-IDS compound over time are presented in Table 8 and Fig. 7.

Table 8. Values of concentrations of the HIR-FAB-IDS compound in blood serum when using different doses of the drug

| Dose/time | 0 h | 3 h | 3.5 h | 4 h | 4.5 h | 5 h | 7 h | 8 h | 27 h |
|---|---|---|---|---|---|---|---|---|---|
| 1 mg/kg | 0.00 | 1919.10 | 1289.04 | 1041.31 | 844.10 | 705.89 | 401.08 | 251.93 | 0.00 |
| 2 mg/kg | 0.00 | 4571.73 | 3184.31 | 2878.93 | 2480.03 | 2353.50 | 1975.85 | 1213.81 | 13.01 |
| 3 mg/kg | 0.00 | 12139.96 | 8650.59 | 6259.27 | 5097.45 | 4217.29 | 2513.06 | 2029.04 | 63.75 |

After administration of the drug at doses of 2 and 3 mg/kg, 2.5 hours after the end of the infusion, the drug was determined in the CSF at concentrations of 25.939 and 272.569 pg/mL, respectively, using the NICO method of 100 pg/mL (Table 9).

Table 9. Concentration of the HIR-FAB-IDS compound in CSF and blood serum after administration of the drug at doses of 2 and 3 mg/kg

| Dose | Time | CSF, pg/mL* | Serum ng/mL** |
|---|---|---|---|
| | Initial, before therapy | (-) | (-) |
| 2 mg/kg | Week 6 | 25.936 | No data |
| 3 mg/kg | Week 10 | 272.569 | 1461.06 |
| * LLOQ for CSF determination 100 pg/mL <br> ** LLOQ for serum determination 50 ng/mL. | | | |

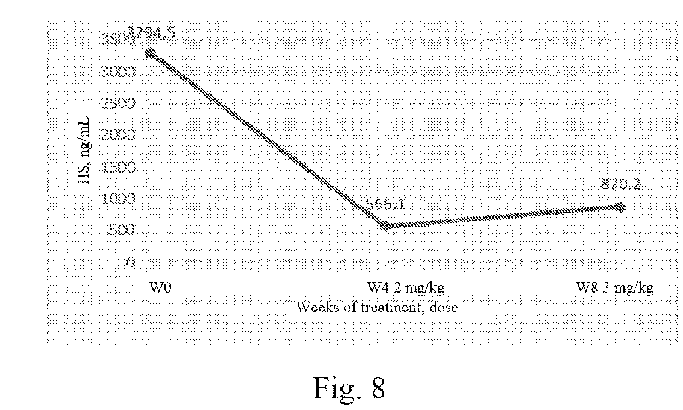

A significant decrease in the accumulation of heparan sulfate (HS) in the CSF was also noted (Fig. 8), which confirms the presence of enzymatic activity of the HIR-FAB-IDS compound in the tissues of the central nervous system and, therefore, indicates the effectiveness of the drug in the treatment of a patient with the neuropathic form of MPS type II.

## Claims

1. A HIR-Fab-IDS compound for the prophylaxis or treatment of a lysosomal enzyme deficiency in a subject having a lysosomal storage disease, comprising a first amino acid sequence of SEQ ID NO:2 and a second amino acid sequence of SEQ ID NO:4.

2. The compound according to claim 1, wherein the lysosomal storage disease is mucopolysaccharidosis type II or mucopolysaccharidosis type II with a neurological component.

3. The compound according to claim 2, wherein the neurological component is represented by a neuropathic form.

4. The compound according to claims 1-3, wherein the subject is a human.

5. Use of the compound according to claim 1 for the prophylaxis or treatment of a lysosomal enzyme deficiency in a subject having a lysosomal storage disease, wherein the subject is administered at least one dose of the HIR-Fab-IDS compound of about 1 to 12 mg/kg.

6. The use according to claim 5, **characterized in that** the dose is selected from a group consisting of about 3 mg/kg, about 6 mg/kg, about 12 mg/kg of the HIR-Fab-IDS compound.

7. The use according to claims 5-6, wherein the lysosomal storage disease is mucopolysaccharidosis type II or mucopolysaccharidosis type II with a neurological component.

8. The use according to claim 7, wherein the neurological component is represented by a neuropathic form.

9. The use according to claims 5-8, wherein the subject is a human.

10. A pharmaceutical composition for the prophylaxis or treatment of a lysosomal enzyme deficiency in a subject having a lysosomal storage disease, wherein the composition comprises a HIR-Fab-IDS compound represented by a first amino acid sequence SEQ ID NO:2 and a second amino acid sequence SEQ ID NO:4.

11. The pharmaceutical composition according to claim 10, wherein the pharmaceutical composition additionally contains sodium chloride, sodium dihydrogen phosphate dihydrate, sodium hydroxide, and polysorbate.

12. The pharmaceutical composition according to claim 11, wherein the pharmaceutical composition additionally contains 5.3 mg of the HIR-Fab-IDS compound, sodium chloride in an amount of 8.0 mg, sodium dihydrogen phosphate dihydrate in an amount of 3.12 mg, sodium hydroxide to adjust pH to pH 6.0, polysorbate 20 in an amount of 0.2 mg, and water for injection up to 1.0 mL.

13. The pharmaceutical composition according to claims 10-11, wherein the lysosomal storage disease is mucopolysaccharidosis type II or mucopolysaccharidosis type II with a neurological component.

14. The pharmaceutical composition according to claim 13, wherein the neurological component is represented by a neuropathic form.

15. The pharmaceutical composition according to claims 10-14, where the subject is a human.

16. Use of the pharmaceutical composition according to claim 10 for the prophylaxis or treatment of a lysosomal enzyme deficiency in a subject having a lysosomal storage disease, wherein the HIR-Fab-IDS compound is administered to the subject at least in one dose selected from a group consisting of about 3 mg/kg, about 6 mg/kg, about 12 mg/kg of the HIR-Fab-IDS compound.

17. A method for prophylaxis or treatment of a lysosomal enzyme deficiency in a subject having a lysosomal storage disease, comprising administering to a patient at least one dose of a HIR-Fab-IDS compound represented by a first amino acid sequence of SEQ ID NO:2 and a second amino acid sequence of SEQ ID NO:4, wherein the dose is selected from a group consisting of about 3 mg/kg, about 6 mg/kg, about 12 mg/kg of the HIR-Fab-IDS compound.

18. The method according to claim 16, wherein the HIR-Fab-IDS compound in the pharmaceutical composition is administered intravenously for 3 hours once a week.

19. The method of claim 16, wherein the lysosomal storage disease is mucopolysaccharidosis type II or mucopolysaccharidosis type II with a neurological component.

20. The method according to claim 18, wherein the neurological component is represented by a neuropathic form.

21. The method according to claims 16-19, wherein the subject is a human.

Fig. 1

Fig. 2

Fig. 3

Fig. 4

Fig. 5

Fig. 6

Fig. 7

Fig. 8

## INTERNATIONAL SEARCH REPORT

| | International application No. |
|---|---|
| | PCT/RU 2024/050043 |

**A.   CLASSIFICATION OF SUBJECT MATTER**

see the supplemental sheet

According to International Patent Classification (IPC) or to both national classification and IPC

**B.   FIELDS SEARCHED**

Minimum documentation searched (classification system followed by classification symbols)

C07K, C12N, A61K, A61P

Documentation searched other than minimum documentation to the extent that such documents are included in the fields searched

Electronic data base consulted during the international search (name of data base and, where practicable, search terms used)

E-Library, Espacenet, PatSearch, PATENTSCOPE, RUPTO, NCBI, EMBL-EBI, Google Scholar, PubMed, USPTO, ScienceDirect

**C.   DOCUMENTS CONSIDERED TO BE RELEVANT**

| Category* | Citation of document, with indication, where appropriate, of the relevant passages | Relevant to claim No. |
|---|---|---|
| A | US 8834874 B2 (ARMAGEN TECHNOLOGIES, INC.), 16.09.2014, the claims, example 4, fig.12, SEQ ID N0: 10 | 1-21 |
| A | US 7741446 B2 (ARMAGEN TECHNOLOGIES, INC.), 22.06.2010, abstract, the claims, SEQ ID N0:29 | 1-21 |
| A | RU 2673038 C2 (OOO MEZHDUNARODNYI BIOTEKHNOLOGICHESKII TSENTR "GENERIUM"), 21.11.2018, abstract, the claims | 1-21 |
| A | RUBEN J. BOADO et al. Insulin Receptor Antibody-Iduronate 2-Sulfatase Fusion Protein: Pharmacokinetics, Anti-Drug Antibody, and Safety Pharmacology in Rhesus Monkeys. Biotechnol Bioeng, 2014 November; 111(11): 2317-2325. doi:10.1002/bit.25289, abstract | 1-21 |

☐  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 May 2024 (06.05.2024) | 20 June 2024 (20.06.2024) |

| Name and mailing address of the ISA/ RU: | Authorized officer |
|---|---|
| | |
| Facsimile No. | Telephone No. |

Form PCT/ISA/210 (second sheet) (July 1998)

**INTERNATIONAL SEARCH REPORT**

International application No.

PCT/RU 2024/050043

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. [X] forming part of the international application as filed.

   b. ☐ furnished subsequent to the international filing date for the purposes of international search (Rule 13*ter* 1(a)),

       ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet (1)) (July 2022)

**INTERNATIONAL SEARCH REPORT**

| International application No. |
| --- |
| PCT/RU 2024/050043 |

A. CLASSIFICATION OF SUBJECT MATTER

**C07K 16/26** (2006.01)
*C12N 9/16* (2006.01)
*A61K 38/46* (2006.01)
*A61K 39/395* (2006.01)
*A61P 3/00* (2006.01)
*A61P 25/28* (2006.01)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8834874 B2
- US 5932211 A
- US 6153188 A
- US 6541254 B1
- US 5208020 A, Chari Ravi J.
- US 04051993 A
- WO 2011044542 A1
- WO 9312227 A
- WO 9203918 A
- WO 9402602 A
- WO 9425585 A
- WO 9634096 A
- WO 9633735 A
- EP 239400 A
- WO 9602576 A
- WO 9951743 A
- WO 2011090088 A
- US 3773919 A
- EP 58481 A
- EP 133988 A

**Non-patent literature cited in the description**

- **MUENZER J. et al.** *Genet. Med.*, August 2006, vol. 8 (8), 465-473
- **AUSUBEL et al.** National Center for Biotechnology Information website. National Institutes of Health website, 1999, 7-58, 7-60
- **SAMBROOK et al.** Molecular Cloning: A Laboratory Manual. CSH Press, Cold Spring Harbor, 1989
- Current Protocols in Molecular Biology. John Wiley & Sons, 2001
- *CHEMICAL ABSTRACTS*, 889958-08-1
- **CHARI R et al.** *Cancer Res.*, 1992, vol. 52, 127-131
- **ZHANG B.** ; **ROTH R.A.** *Proc. Natl. Acad. Sci. USA.*, 1991, vol. 88 (21), 9858-9862
- **S. A. PRIGENT** ; **K. K, STANLEY** ; **K, SIDDLE**. *J. Biol.*, 1990
- **YIP et al.** *J. Biol. Chem.*, 2003, vol. 278 (30), 27329-27332
- **WHITTAKER et al.** *J Biol. Chem.*, 2005, vol. 280 (22), 20932-20936
- **KASUYA et al.** *Biochemistry*, 1993, vol. 32, 13531-13536
- Remington's Pharmaceutical Sciences. 1980
- **FELICE B.R.** ; **WRIGHT T.L** ; **BOYD R.B**. Safety Evaluation of Chronic Intrathecal Administration of Idursulfase-GG in Cynomolgus Monkeys. *Toxicol. Pathol.*, August 2011, vol. 39 (5), 879-9
- **DAVID L. VALLE** ; **STYLIANOS ANTONARAKIS** ; **ANDREA BALLABIO** ; **ARTHUR L. BEAUDET** ; **GRANT A. MITCHELL**. *McGraw-Hill Education*, 2007
- **WANG et al.** *Molecular Genetics and Metabolism*, 2009
- **RALUY-CALLADO M. et al.** *Orphanet. J. Rare Dis.*, 2013, vol. 8, 101
- **ELSA G. SHAPIRO** ; **JULIE B. EISENGART**. The natural history of neurocognition in MPS disorders: a review. *Molecular Genetics and Metabolism*, 2021, vol. 133 (1), 8-34
- **S. AL SAWAF** ; **E. MAYATEPEK** ; **B. HOFFMANN**. Neurological findings in Hunter disease:pathology and possible therapeutic effects reviewed. *J. Inherit. Metab. Dis.*, 2008, vol. 31 (4), 473-480
- **I. V. SCHWARTZ et al.** A clinical study of 77 patients with mucopolysaccharidosis type II. *Acta Paediatr.*, 2007, vol. 96 (455), 63-70
- **SATO K. et al.** *Cancer Research*, 1993, vol. 53, 851-856
- **SEE BROWN T**. Gene Cloning. Chapman & Hall, 1995
- **WATSON R et al.** Recombinant DNA. Scientific American Books, 1992
- **ALBERTS B et al.** Molecular Biology of the Cell. Garland Publishing Inc., 2008
- PCR Protocols. A Guide to Methods and Applications. Academic Press Inc., 1990
- PCR Technology. Principles and Applications for DNA Amplification. Stockton Press, 1989
- **SAMBROOK J et al.** Molecular Cloning. A Laboratory Manual. Cold Spring Harbor Laboratory Press, 1989
- **BISHOP T et al.** Nucleic Acid and Protein Sequence. A Practical Approach. IRL Press, 1987
- Maximizing Gene Expression. Butterworths Publishers, 1987
- **DAVIS L et al.** Basic Methods in Molecular Biology. Elsevier Science Publishing Co., 1986

- Plasmid for Therapy and Vaccination. Wiley-VCH Verlag GmbH, 2001
- *Expert Opin. Drug Deliv.*, July 2007, vol. 4 (4), 427-440
- Remington's Pharmaceutical Science. 1980
- **LANGER et al.** *J. Biomed. Mater. Res.*, 1981, vol. 15, 267-277
- **LANGER**. *Chemtech.*, 1982, vol. 12, 98-105
- **SIDMAN et al.** *Biopolymers*, 1983, vol. 22, 547-556
- **TANAKA N.** ; **KIDA S.** ; **KINOSHITA M.** ; **MORIMOTO H.** ; **SHIBASAKI T.** ; **TACHIBANA K.** ; **YAMAMOTO R**. Evaluation of cerebrospinal fluid heparan sulfate as a biomarker of neuropathology in a murine model of mucopolysaccharidosis type II using high-sensitivity LC/MS/MS. *Mol. Genet. Metab*, September 2018, vol. 125 (1-2), 53-58